# EUROPEAN PATENT APPLICATION

(11) **EP 1 239 036 A1**
(43) Date of publication of application: **11.09.2002**
(21) Application number: 00981683.6
(22) Date of filing: 13.12.2000
(51) Int. Cl.: C12N 15/12, C12N 15/63, C12N 7/01, C07K 14/47, C07K 16/18, C12N 5/12, C12Q 1/68, A61K 38/17, A61P 35/00, C12M 1/00, C12P 21/02

(54) **POLYNUCLEOTIDES, POLYPEPTIDES, REMEDIES FOR CANCER AND VACCINES**

(30) Priority: 13.12.1999 JP 35368899
(71) Applicant: Japan Tissue Engineering Co., Ltd., Gamagori-shi, Aichi 443-0022 (JP); Abe, Akihiro, Nagoya-shi, Aichi 466-0051 (JP)
(72) Inventor: ABE, Akihiro, Nagoya-shi, Aichi 466-0051 (JP); EMI, Nobuhiko, Nagoya-shi, Aichi 466-0804 (JP); SAITO, Hidehiko, Nagoya-shi, Aichi 466-0811 (JP)
(74) Representative: Reinhard - Skuhra - Weise & Partner
(86) International application number: JP0008813
(87) International publication number: WO01042460

(57) **Abstract**

The present invention provides polynucleotides, polypeptides, remedies for cancer, and vaccines, which are used for diagnosing autoimmune diseases such as graft-versus-host disease, and graft-versus-leukemia, and treating and preventing acute or chronic myeloid leukemia. The polynucleotides includes a base sequence that is identical or substantially identical with a base sequence represented by SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, and SEQ ID NO: 10. The polypeptide includes an amino acid sequence that is identical or substantially identical with an amino acid sequence encoded by the above-mentioned polynucleotide. The remedy for cancer contains the polynucleotides or polypeptides, and strengthens the defense mechanism in a living body to thereby treat cancer. The vaccine contains the polynucleotides or polypeptides, and makes living bodies to acquire the defense mechanism to thereby prevent the onset of cancer.

SEQUENCE LISTING

<110> ABE, Akihiro
      JAPAN TISSUE ENGINEERING CO., LTD.

<120> Polynucleotides, polypeptides, remedies for cancer and vaccines

<130> P3P2000249

<140>
<141>

<150> JP 11-353688
<151> 1999-12-13

<160> 10

<170> PatentIn Ver. 2.1

<210> 1
<211> 120
<212> DNA
<213> Homo sapiens

<220>
<221> CDS
<222> (1)..(120)

<400> 1
cag aca aaa tcc tgt cag tcc cag ccc tcc caa act aaa cct tct cca     48
Gln Thr Lys Ser Cys Gln Ser Gln Pro Ser Gln Thr Lys Pro Ser Pro
  1               5                  10                  15

tgc aaa tct act cag cct aag cca agc cag ccc tgg cct ccc cag tct     96
Cys Lys Ser Thr Gln Pro Lys Pro Ser Gln Pro Trp Pro Pro Gln Ser
             20                  25                  30

aag cct tct cag ccc aga ccc cct                                     120
Lys Pro Ser Gln Pro Arg Pro Pro
         35                  40

<210> 2
<211> 3984
<212> DNA
<213> Homo sapiens

<220>
<221> CDS
<222> (1)..(3984)

<400> 2
agt tta tca gaa gaa cat cag ccc ttt tct gga aaa act gca tat ctg     48
Ser Leu Ser Glu Glu His Gln Pro Phe Ser Gly Lys Thr Ala Tyr Leu
1               5                   10                  15

ttt tcc cca gac cac tca gat gag aaa cta ata gaa aaa gaa aat caa     96
Phe Ser Pro Asp His Ser Asp Glu Lys Leu Ile Glu Lys Glu Asn Gln
            20                  25                  30

att gat aca gca ttt tta tct agc act agt aaa tat gaa aag ctt gaa     144
Ile Asp Thr Ala Phe Leu Ser Ser Thr Ser Lys Tyr Glu Lys Leu Glu
        35                  40                  45

aaa cat tca gca aat cat aat gtt aaa gat gca act aaa gaa aac agt     192
Lys His Ser Ala Asn His Asn Val Lys Asp Ala Thr Lys Glu Asn Ser
    50                  55                  60

tgt gac gct aat gaa gta ata aat gaa agt aat tct gta tct tta agt     240
Cys Asp Ala Asn Glu Val Ile Asn Glu Ser Asn Ser Val Ser Leu Ser
65                  70                  75                  80

tgc ata aaa gaa aac ata aat tct agt aca ggc aac gat tgt gat gca     288
Cys Ile Lys Glu Asn Ile Asn Ser Ser Thr Gly Asn Asp Cys Asp Ala
                85                  90                  95

act tgc ata ggt cac aca aag gcg aaa act gac gta ctt ata tca gtc     336
Thr Cys Ile Gly His Thr Lys Ala Lys Thr Asp Val Leu Ile Ser Val
            100                 105                 110

tta gat tca aat gtg aag cac ttt tta aat gat ctc tac caa caa ggt     384
Leu Asp Ser Asn Val Lys His Phe Leu Asn Asp Leu Tyr Gln Gln Gly
        115                 120                 125

aac ctt att tta tct gat tgt aaa aga aac ctg gaa gta aag tgg aca 432
Asn Leu Ile Leu Ser Asp Cys Lys Arg Asn Leu Glu Val Lys Trp Thr
    130                 135                 140

gat cct att gag aga ccc ata caa aac att att aca gga aac ttc ctt 480
Asp Pro Ile Glu Arg Pro Ile Gln Asn Ile Ile Thr Gly Asn Phe Leu
145                 150                 155                 160

atg ggc cca tta aac cta act ttg ata gca agt aaa aag tac agt att 528
Met Gly Pro Leu Asn Leu Thr Leu Ile Ala Ser Lys Lys Tyr Ser Ile
                165                 170                 175

cct cag tta tca gcc gct gca gtg aca gat agt gag gga gaa tct tca 576
Pro Gln Leu Ser Ala Ala Ala Val Thr Asp Ser Glu Gly Glu Ser Ser
            180                 185                 190

aaa tct tac ttg gat aag cag aga att ctt act gta gat tct ttt gca 624
Lys Ser Tyr Leu Asp Lys Gln Arg Ile Leu Thr Val Asp Ser Phe Ala
        195                 200                 205

gca tcc agt act gta cca cac tgt gag cag agc tgt aga gaa aaa gag 672
Ala Ser Ser Thr Val Pro His Cys Glu Gln Ser Cys Arg Glu Lys Glu
    210                 215                 220

ctt cta aag aca gaa cag tgc tct tca ggt aat tgc ctc cat aca gat 720
Leu Leu Lys Thr Glu Gln Cys Ser Ser Gly Asn Cys Leu His Thr Asp
225                 230                 235                 240

ggg aat gaa aca aat gtc act gag aat tat gag ttg gat gta gca tca 768
Gly Asn Glu Thr Asn Val Thr Glu Asn Tyr Glu Leu Asp Val Ala Ser
                245                 250                 255

gga act gaa gaa gat aaa agt tat ggg gaa aat ata gtg gaa tta tct 816
Gly Thr Glu Glu Asp Lys Ser Tyr Gly Glu Asn Ile Val Glu Leu Ser
            260                 265                 270

tcc agt gat agt tct ctg ctt tta aaa gat aat gta aaa ggc tcc tct 864
Ser Ser Asp Ser Ser Leu Leu Leu Lys Asp Asn Val Lys Gly Ser Ser
        275                 280                 285

tca gaa aca tgt att gtg aag aaa gac act gag gac aga ata acg tgg 912

Ser Glu Thr Cys Ile Val Lys Lys Asp Thr Glu Asp Arg Ile Thr Trp
    290                 295                 300

aaa gtt aaa caa gcg gaa aaa gca aaa gat tct gtt tac aaa aga agc 960
Lys Val Lys Gln Ala Glu Lys Ala Lys Asp Ser Val Tyr Lys Arg Ser
305                 310                 315                 320

atg act gaa gga tca act gtt aat act gag tac aaa aat caa aag aat 1008
Met Thr Glu Gly Ser Thr Val Asn Thr Glu Tyr Lys Asn Gln Lys Asn
                325                 330                 335

cag atc tca gaa gaa tcc tgc tta aat gag aaa att att aca act aac 1056
Gln Ile Ser Glu Glu Ser Cys Leu Asn Glu Lys Ile Ile Thr Thr Asn
            340                 345                 350

ttg att gat tcc cat ctg agc act aaa aat act acc act gag tca gtc 1104
Leu Ile Asp Ser His Leu Ser Thr Lys Asn Thr Thr Thr Glu Ser Val
        355                 360                 365

cct ttg aag aac aca gtt tct aat ccg ctt aac aaa aga gag aag aac 1152
Pro Leu Lys Asn Thr Val Ser Asn Pro Leu Asn Lys Arg Glu Lys Asn
    370                 375                 380

ccc caa att aaa gtt agt aaa gac tcg cag tct gac ttg aca tta cat 1200
Pro Gln Ile Lys Val Ser Lys Asp Ser Gln Ser Asp Leu Thr Leu His
385                 390                 395                 400

tca gaa ata gcc tat att tcc aaa cca gga att cta gga gtt aat cat 1248
Ser Glu Ile Ala Tyr Ile Ser Lys Pro Gly Ile Leu Gly Val Asn His
                405                 410                 415

acg cct att tta cct gcc cac tct gaa acc tgt aaa gtc cct act ctt 1296
Thr Pro Ile Leu Pro Ala His Ser Glu Thr Cys Lys Val Pro Thr Leu
            420                 425                 430

ctg aag aaa cct gcg tca tac gtg agt gat ttt aaa gaa aaa cat tgc 1344
Leu Lys Lys Pro Ala Ser Tyr Val Ser Asp Phe Lys Glu Lys His Cys
        435                 440                 445

tca gct aat cat acg gcc ctt ata gct aat cta tct caa att ttg cag 1392
Ser Ala Asn His Thr Ala Leu Ile Ala Asn Leu Ser Gln Ile Leu Gln
    450                 455                 460

agg gca gat gaa gca tca tct ttg cag att cta cag gaa gaa act aag 1440
Arg Ala Asp Glu Ala Ser Ser Leu Gln Ile Leu Gln Glu Glu Thr Lys
465                 470                 475                 480

gtt tgt cta aat att ctc cct tta ttt gtg gaa gct ttt gaa aga aag 1488
Val Cys Leu Asn Ile Leu Pro Leu Phe Val Glu Ala Phe Glu Arg Lys
                485                 490                 495

caa gaa tgt tca gtt gaa caa atc ctg att tca aga gaa ctg ttg gta 1536
Gln Glu Cys Ser Val Glu Gln Ile Leu Ile Ser Arg Glu Leu Leu Val
            500                 505                 510

gac caa aac ctg tgg aat aat tgc aaa cac aca tta aaa cca tgt gct 1584
Asp Gln Asn Leu Trp Asn Asn Cys Lys His Thr Leu Lys Pro Cys Ala
        515                 520                 525

gtt gac act ttg gta gaa ctt caa atg atg atg gaa aca att caa ttc 1632
Val Asp Thr Leu Val Glu Leu Gln Met Met Met Glu Thr Ile Gln Phe
    530                 535                 540

att gaa aac aaa aaa agg cac tta gaa ggt gaa cca aca ttg cga agc 1680
Ile Glu Asn Lys Lys Arg His Leu Glu Gly Glu Pro Thr Leu Arg Ser

545                 550                 555                 560

ttg ctt tgg tat gat gaa aca ctg tat gct gag ctt ctt gga aaa cca 1728
Leu Leu Trp Tyr Asp Glu Thr Leu Tyr Ala Glu Leu Leu Gly Lys Pro
                565                 570                 575

cgt gga ttt caa cag cag tct aat ttc tat cct ggt ttc caa gga aga 1776
Arg Gly Phe Gln Gln Gln Ser Asn Phe Tyr Pro Gly Phe Gln Gly Arg
            580                 585                 590

tta aaa tat aat gca ttc tgt gag tta cag act tac cat gat caa tta 1824
Leu Lys Tyr Asn Ala Phe Cys Glu Leu Gln Thr Tyr His Asp Gln Leu
        595                 600                 605

gtt gaa ttg ctt gag gaa aca aaa agg gaa aag aat tca tac tat gta 1872
Val Glu Leu Leu Glu Glu Thr Lys Arg Glu Lys Asn Ser Tyr Tyr Val
    610                 615                 620

ttc tta aag tac aaa cga cag gtt aat gaa tgt gaa gcc ata atg gag 1920
Phe Leu Lys Tyr Lys Arg Gln Val Asn Glu Cys Glu Ala Ile Met Glu
625                 630                 635                 640

cat tgt tcc gat tgc ttt gat ttt tct ctt tct gtt cca ttt acc tgt 1968
His Cys Ser Asp Cys Phe Asp Phe Ser Leu Ser Val Pro Phe Thr Cys
                645                 650                 655

gga gtt aac ttt gga gat agt tta gaa gac ctg gaa atc tta aga aaa 2016
Gly Val Asn Phe Gly Asp Ser Leu Glu Asp Leu Glu Ile Leu Arg Lys
            660                 665                 670

agt act tta aag ttg atc aat gta tgt ggg gac tct cct aaa gtt cat 2064
Ser Thr Leu Lys Leu Ile Asn Val Cys Gly Asp Ser Pro Lys Val His
        675                 680                 685

tcg tat cca gga aaa cag gac cat ctg tgg att atc ata gaa atg atc 2112
Ser Tyr Pro Gly Lys Gln Asp His Leu Trp Ile Ile Ile Glu Met Ile
    690                 695                 700

tcc tca aag gtt aat ttt att aag aac aac gag gca gta cgt gtt aaa 2160
Ser Ser Lys Val Asn Phe Ile Lys Asn Asn Glu Ala Val Arg Val Lys
705                 710                 715                 720

ata tct ctt tat ggt ctg gaa cat atc ttt ttt gat gct gca aaa aat 2208
Ile Ser Leu Tyr Gly Leu Glu His Ile Phe Phe Asp Ala Ala Lys Asn
                725                 730                 735

ctt gtt tgg aaa gag aga aca caa tcc ttc agc aaa aaa tac tca caa 2256
Leu Val Trp Lys Glu Arg Thr Gln Ser Phe Ser Lys Lys Tyr Ser Gln
            740                 745                 750

aag aag gac gaa gaa agg cta ctc aga gtg aat aaa tgt gcc ttt tct 2304
Lys Lys Asp Glu Glu Arg Leu Leu Arg Val Asn Lys Cys Ala Phe Ser
        755                 760                 765

aag ttg cag aag ata tat gat act ttg tct aaa gat tta aac aat gaa 2352
Lys Leu Gln Lys Ile Tyr Asp Thr Leu Ser Lys Asp Leu Asn Asn Glu
    770                 775                 780

cca att tcc cct att ggg ctt gag gag gat act ata att gct tcc aga 2400
Pro Ile Ser Pro Ile Gly Leu Glu Glu Asp Thr Ile Ile Ala Ser Arg

785                 790                 795                 800

aag tca gat cat cca ata acg aag cac att agc ata gaa aat tct aaa     2448
Lys Ser Asp His Pro Ile Thr Lys His Ile Ser Ile Glu Asn Ser Lys
                805                 810                 815

ttt aac agt aat ttg ctt gca cac cca gat att tgt tgt att agt gag     2496
Phe Asn Ser Asn Leu Leu Ala His Pro Asp Ile Cys Cys Ile Ser Glu
            820                 825                 830

ata ttg gat cag gct gaa ttt gca gac ctt aaa aaa tta cag gat ctc     2544
Ile Leu Asp Gln Ala Glu Phe Ala Asp Leu Lys Lys Leu Gln Asp Leu
        835                 840                 845

acc ttg aga tgt aca gat cac tta gaa att tta aaa aaa tac ttt cag     2592
Thr Leu Arg Cys Thr Asp His Leu Glu Ile Leu Lys Lys Tyr Phe Gln
    850                 855                 860

atg cta caa gat aat aac atg gat aat att ttt atc aca gaa gaa aat     2640
Met Leu Gln Asp Asn Asn Met Asp Asn Ile Phe Ile Thr Glu Glu Asn
865                 870                 875                 880

gtt tta gac gtg gtg ata aac cac agc cat gag gct atc att tta aag     2688
Val Leu Asp Val Val Ile Asn His Ser His Glu Ala Ile Ile Leu Lys
                885                 890                 895

cct gaa gct att gaa atg tat att gaa atc gtc atg gtc tca gaa aca     2736
Pro Glu Ala Ile Glu Met Tyr Ile Glu Ile Val Met Val Ser Glu Thr
            900                 905                 910

att cac ttt ctt aaa aac tca ata gca aag aaa cta gac aaa cag agg     2784
Ile His Phe Leu Lys Asn Ser Ile Ala Lys Lys Leu Asp Lys Gln Arg
        915                 920                 925

ttt cga ggt atg ctt tgg ttt gat ttg tca ctt ctt cct gag ctg gtt     2832
Phe Arg Gly Met Leu Trp Phe Asp Leu Ser Leu Leu Pro Glu Leu Val
    930                 935                 940

cag tgc caa gaa aaa atg gct tct ttt tca ttt ctt aaa gat aac tca     2880
Gln Cys Gln Glu Lys Met Ala Ser Phe Ser Phe Leu Lys Asp Asn Ser
945                 950                 955                 960

aca gat gtt tgc ctt tgg aaa gtg ata gag act gct gtt tcc gaa ctt 2928
Thr Asp Val Cys Leu Trp Lys Val Ile Glu Thr Ala Val Ser Glu Leu
                965                 970                 975

aag aaa gat ctg gat att atc tgg caa aat ata atg aaa gct gtt aaa 2976
Lys Lys Asp Leu Asp Ile Ile Trp Gln Asn Ile Met Lys Ala Val Lys
            980                 985                 990

ttt gct cat atg cta ttc att tgc ttc ttc aag gag aac ttc aag gaa 3024
Phe Ala His Met Leu Phe Ile Cys Phe Phe Lys Glu Asn Phe Lys Glu
        995                 1000                1005

cct ttc aga aat aaa aaa gct tct gaa gaa gtc caa gta tta att cca 3072
Pro Phe Arg Asn Lys Lys Ala Ser Glu Glu Val Gln Val Leu Ile Pro
    1010                1015                1020

cat ata ttg acc ttt gtg cca tat ata gca tcc ata aat tat gga agc 3120
His Ile Leu Thr Phe Val Pro Tyr Ile Ala Ser Ile Asn Tyr Gly Ser
1025                1030                1035                1040

act gtg aca gag tta gaa tac aac tac aat caa ttt tct aca ctg ctg 3168
Thr Val Thr Glu Leu Glu Tyr Asn Tyr Asn Gln Phe Ser Thr Leu Leu
                1045                1050                1055

aag aat gta atg tct gcc cct agg aaa gat tta gga aaa atg gcc cac 3216
Lys Asn Val Met Ser Ala Pro Arg Lys Asp Leu Gly Lys Met Ala His
            1060                1065                1070

att agg aaa gtc atg aaa acg att gaa cat atg aag atg ata tgt act 3264
Ile Arg Lys Val Met Lys Thr Ile Glu His Met Lys Met Ile Cys Thr
        1075                1080                1085

aaa aat gct gaa cta acc att tcc ttt ttc cta tgc caa atg ctg tat 3312
Lys Asn Ala Glu Leu Thr Ile Ser Phe Phe Leu Cys Gln Met Leu Tyr
    1090                1095                1100

aac aga agg aag att tta cag ctg aag aga aaa gaa aaa atg aat att 3360
Asn Arg Arg Lys Ile Leu Gln Leu Lys Arg Lys Glu Lys Met Asn Ile
1105                1110                1115                1120

cat att gta aaa cct ggg gaa aat aac aat aaa ttt agt att tct acg 3408
His Ile Val Lys Pro Gly Glu Asn Asn Asn Lys Phe Ser Ile Ser Thr

1125                1130                1135

atg ttg ccc cca gta tca gag tgc ata aac aaa aac atc tca aat tcc    3456
Met Leu Pro Pro Val Ser Glu Cys Ile Asn Lys Asn Ile Ser Asn Ser
        1140                1145                1150

tct aaa aaa cga ccg agc act gta gac aaa tgt gaa gac tct cag gaa    3504
Ser Lys Lys Arg Pro Ser Thr Val Asp Lys Cys Glu Asp Ser Gln Glu
    1155                1160                1165

cag cag caa gat act act gtt tcc agt tgt aaa aag cta aag gta gac    3552
Gln Gln Gln Asp Thr Thr Val Ser Ser Cys Lys Lys Leu Lys Val Asp
1170                1175                1180

atg aaa gat gtc aca aaa atc aac aga gaa aag gca aca ttc aag cat    3600
Met Lys Asp Val Thr Lys Ile Asn Arg Glu Lys Ala Thr Phe Lys His
1185                1190                1195                1200

cca agg act aca gga tct cat ccc aaa agc gaa aac aaa ata gta cca    3648
Pro Arg Thr Thr Gly Ser His Pro Lys Ser Glu Asn Lys Ile Val Pro
                1205                1210                1215

agt tca tgt gac agt ctg aaa aga aat cat tta acg cca aaa aag gtt    3696
Ser Ser Cys Asp Ser Leu Lys Arg Asn His Leu Thr Pro Lys Lys Val
            1220                1225                1230

gaa atg caa aga tca cta cct ggc tca ctt tta ccc tta gag aac cca    3744
Glu Met Gln Arg Ser Leu Pro Gly Ser Leu Leu Pro Leu Glu Asn Pro
        1235                1240                1245

aaa gac act tgc gca tca aag tcg gaa agc aaa ata gac tta act gtt    3792
Lys Asp Thr Cys Ala Ser Lys Ser Glu Ser Lys Ile Asp Leu Thr Val
    1250                1255                1260

tca tct gat cac ttc agt gga caa cag gaa aat tta aat agc atg aag    3840
Ser Ser Asp His Phe Ser Gly Gln Gln Glu Asn Leu Asn Ser Met Lys
1265                1270                1275                1280

aaa aga aat gtg aac ttc agt gct gct gaa aca aaa agt gat aag aaa    3888
Lys Arg Asn Val Asn Phe Ser Ala Ala Glu Thr Lys Ser Asp Lys Lys
                1285                1290                1295

gat tgt gct gct ttt gca att tgt gac caa aaa agt gta cat ggc aca    3936
Asp Cys Ala Ala Phe Ala Ile Cys Asp Gln Lys Ser Val His Gly Thr
            1300                1305                1310

ttt tca cca gac cat ggg aca ctt ttg cag aaa ttt ctt aaa aat tcc    3984
Phe Ser Pro Asp His Gly Thr Leu Leu Gln Lys Phe Leu Lys Asn Ser
        1315                1320                1325

<210> 3
<211> 409
<212> DNA
<213> Homo sapiens

<220>
<221> CDS
<222> (1)..(351)

<400> 3
   agc ttt tgc agg gcc aac cag gct gcc cgc cac gtg tgc ctc aac cag    48
   Ser Phe Cys Arg Ala Asn Gln Ala Ala Arg His Val Cys Leu Asn Gln
   1               5                   10                  15
   agc atc gac act tac acc atg gtg gac aaa cag act ctg gaa ctc tgc    96
   Ser Ile Asp Thr Tyr Thr Met Val Asp Lys Gln Thr Leu Glu Leu Cys
               20                  25                  30
   aca ttt gaa gaa ggg agt cag atg gac aac atg ctg gtg caa aca aac    144
   Thr Phe Glu Glu Gly Ser Gln Met Asp Asn Met Leu Val Gln Thr Asn
           35                  40                  45
   aaa ccc tac aaa tgc aac ttg tgt gac aaa aca ttc tcc act ccc aat    192
   Lys Pro Tyr Lys Cys Asn Leu Cys Asp Lys Thr Phe Ser Thr Pro Asn
       50                  55                  60
   gag gtt gtt aaa cat tca tgc caa aac cag aac tcg gat gtt ttt gcc    240
   Glu Val Val Lys His Ser Cys Gln Asn Gln Asn Ser Asp Val Phe Ala
   65                  70                  75                  80
   cta gac gaa ggg cga tcc att ctc ctg ggc agt ggg gac tcg gaa gta    288
   Leu Asp Glu Gly Arg Ser Ile Leu Leu Gly Ser Gly Asp Ser Glu Val
                   85                  90                  95
   acg gag cct gac cac cca gtg tta gct tcc atc aaa aag gaa caa gaa    336
   Thr Glu Pro Asp His Pro Val Leu Ala Ser Ile Lys Lys Glu Gln Glu
               100                 105                 110
   acc gtc tta cta gac tgaatgtcac ttatcttttt aaaaaactct catttttaca    391
   Thr Val Leu Leu Asp
           115
   aagactatct tccctccc                                                 409

<210> 4
<211> 607
<212> DNA
<213> Homo sapiens

<220>
<221> CDS
<222> (1)..(606)

<400> 4
gga agg gaa att cgt gaa ttg aaa gaa ctt cag gat gaa gga aga agt    48
Gly Arg Glu Ile Arg Glu Leu Lys Glu Leu Gln Asp Glu Gly Arg Ser
1               5                   10                  15

gca cga ttc agt gtc ctc agt gtg tgt ttg gta cca att gcc cta aaa    96
Ala Arg Phe Ser Val Leu Ser Val Cys Leu Val Pro Ile Ala Leu Lys
            20                  25                  30

cat ttg tgc aac atg cta aaa ccc atg aaa aag ata aaa ggt act act    144
His Leu Cys Asn Met Leu Lys Pro Met Lys Lys Ile Lys Gly Thr Thr
        35                  40                  45

gct gtg aag agt gaa ctt cag gca gtg aca gaa aat gaa ttg gaa tgc    192
Ala Val Lys Ser Glu Leu Gln Ala Val Thr Glu Asn Glu Leu Glu Cys
    50                  55                  60

cat cga ggc att gca cat ggg gca gtg gta aaa tgc cct atg gtc act    240
His Arg Gly Ile Ala His Gly Ala Val Val Lys Cys Pro Met Val Thr
65                  70                  75                  80

tct gat att gcc cag aga aaa aca caa aaa aag act ttc atg aaa gac     288
Ser Asp Ile Ala Gln Arg Lys Thr Gln Lys Lys Thr Phe Met Lys Asp
                    85                  90                  95

tct gta gta gga tca tcc aaa aaa tca gct acc tac ata tgt aag atg     336
Ser Val Val Gly Ser Ser Lys Lys Ser Ala Thr Tyr Ile Cys Lys Met
                100                 105                 110

tgt cct ttt act act tca gcc aaa agt gtt tta aaa aag cac acg gag     384
Cys Pro Phe Thr Thr Ser Ala Lys Ser Val Leu Lys Lys His Thr Glu
        115                 120                 125

tac ttg cat tca tca tca tgt gtt gat tca ttt ggt agt cct ctt gga     432
Tyr Leu His Ser Ser Ser Cys Val Asp Ser Phe Gly Ser Pro Leu Gly
    130                 135                 140

ctt gat aaa aga aaa aat gac atc ctt gaa gaa cct gta gat agt gat     480
Leu Asp Lys Arg Lys Asn Asp Ile Leu Glu Glu Pro Val Asp Ser Asp
145                 150                 155                 160

agc act aaa aca tta act aaa caa cag tca acc aca ttt cca aag aac     528
Ser Thr Lys Thr Leu Thr Lys Gln Gln Ser Thr Thr Phe Pro Lys Asn
                165                 170                 175

tct gct tta aaa caa gat gtg aag cga aca ttt gga tca acc tca caa     576
Ser Ala Leu Lys Gln Asp Val Lys Arg Thr Phe Gly Ser Thr Ser Gln
            180                 185                 190

tca agt agt ttt tca aaa att cat aag cgg c                           607
Ser Ser Ser Phe Ser Lys Ile His Lys Arg
        195                 200

<210> 5
<211> 398
<212> DNA
<213> Homo sapiens

<220>
<221> CDS
<222> (34)..(396)

<400> 5
tgggaccccg cgcgggctgg ttcgggatga gcg atg gca tcg gtc aag gtg gcc 54
Met Ala Ser Val Lys Val Ala
1               5

gtg agg gtc cgg ccc atg aat cgc agg gaa aag gac ttg gag gcc aag 102
Val Arg Val Arg Pro Met Asn Arg Arg Glu Lys Asp Leu Glu Ala Lys
        10                  15                  20

ttc att att cag atg gag aaa agc aaa acg aca atc aca aac tta aag 150
Phe Ile Ile Gln Met Glu Lys Ser Lys Thr Thr Ile Thr Asn Leu Lys
    25                  30                  35

ata cca gaa gga ggc act ggg gac tca gga aga gaa cgg acc aag acc 198
Ile Pro Glu Gly Gly Thr Gly Asp Ser Gly Arg Glu Arg Thr Lys Thr
40                  45                  50                  55

ttc acc tat gac ttt tct ttt tat tct gct gat aca aaa agc cca gat 246
Phe Thr Tyr Asp Phe Ser Phe Tyr Ser Ala Asp Thr Lys Ser Pro Asp
                60                  65                  70

tac gtt tca caa gaa atg gtt ttc aaa acc ctc ggc aca gat gtc gtg 294
Tyr Val Ser Gln Glu Met Val Phe Lys Thr Leu Gly Thr Asp Val Val
            75                  80                  85

aag tct gca ttt gaa ggt tat aat gct tgt gtc ttt gca tat ggg caa 342
Lys Ser Ala Phe Glu Gly Tyr Asn Ala Cys Val Phe Ala Tyr Gly Gln
        90                  95                  100

act gga tct gga aag tca tac act atg atg gga aat tct gga gat tct 390
Thr Gly Ser Gly Lys Ser Tyr Thr Met Met Gly Asn Ser Gly Asp Ser
    105                 110                 115

ggc tta at 398
Gly Leu
120

<210> 6

<211> 2567
<212> DNA

<213> Homo sapiens

<220>
<221> CDS
<222> (1)..(1122)

<400> 6
gta gaa act gag gag gca gga gta gtg aca aca gca aca gca tct gtt    48
Val Glu Thr Glu Glu Ala Gly Val Val Thr Thr Ala Thr Ala Ser Val
1               5                   10                  15

aat cta aaa gtg agt cct aaa aga gga cga cct gca gct aca gaa gtc    96
Asn Leu Lys Val Ser Pro Lys Arg Gly Arg Pro Ala Ala Thr Glu Val
            20                  25                  30

aag att cca aaa cca aga ggc aga ccc aaa atg gta aaa cag ccc tgt    144
Lys Ile Pro Lys Pro Arg Gly Arg Pro Lys Met Val Lys Gln Pro Cys
        35                  40                  45

cct tca gag agt gac atc att act gaa gag gac aaa agt aag aaa aag    192
Pro Ser Glu Ser Asp Ile Ile Thr Glu Glu Asp Lys Ser Lys Lys Lys
    50                  55                  60

ggg caa gag gaa aaa caa cct aaa aag cag cct aag aag gat gaa gag    240
Gly Gln Glu Glu Lys Gln Pro Lys Lys Gln Pro Lys Lys Asp Glu Glu
65                  70                  75                  80

ggc cag aag gaa gaa gat aag cca aga aaa gag ccg gat aaa aaa gag    288
Gly Gln Lys Glu Glu Asp Lys Pro Arg Lys Glu Pro Asp Lys Lys Glu
                85                  90                  95

ggg aag aaa gaa gtt gaa tca aaa agg aaa aat tta gct aaa aca ggg    336
Gly Lys Lys Glu Val Glu Ser Lys Arg Lys Asn Leu Ala Lys Thr Gly
            100                 105                 110

gtt act tca acc tcc gat tct gaa gaa gaa gga gat gat caa gaa ggt    384
Val Thr Ser Thr Ser Asp Ser Glu Glu Glu Gly Asp Asp Gln Glu Gly
        115                 120                 125

gaa aag aag aga aaa ggt ggg agg aac ttt cag act gct cac aga agg    432
Glu Lys Lys Arg Lys Gly Gly Arg Asn Phe Gln Thr Ala His Arg Arg
    130                 135                 140

aat atg ctg aaa ggc caa cat gag aaa gaa gca gca gat cga aaa cgc 480
Asn Met Leu Lys Gly Gln His Glu Lys Glu Ala Ala Asp Arg Lys Arg
145                 150                 155                 160

aag caa gag gaa caa atg gaa act gag cag cag aat aaa gat gaa gga 528
Lys Gln Glu Glu Gln Met Glu Thr Glu Gln Gln Asn Lys Asp Glu Gly
                165                 170                 175

aag aag cca gaa gtt aag aaa gtg gag aag aag cga gaa aca tca atg 576
Lys Lys Pro Glu Val Lys Lys Val Glu Lys Lys Arg Glu Thr Ser Met
            180                 185                 190

gat tct cga ctt caa agg ata cat gct gag att aaa aat tca ctc aaa 624
Asp Ser Arg Leu Gln Arg Ile His Ala Glu Ile Lys Asn Ser Leu Lys
        195                 200                 205

att gat aat ctt gat gtg aac aga tgc att gag gcc ttg gat gaa ctt 672
Ile Asp Asn Leu Asp Val Asn Arg Cys Ile Glu Ala Leu Asp Glu Leu
    210                 215                 220

gct tca ctt cag gtc aca atg caa caa gct cag aaa cac aca gag atg 720
Ala Ser Leu Gln Val Thr Met Gln Gln Ala Gln Lys His Thr Glu Met
225                 230                 235                 240

att act aca ctg aaa aaa ata cgg cga ttc aaa gtt agt cag gta atc 768
Ile Thr Thr Leu Lys Lys Ile Arg Arg Phe Lys Val Ser Gln Val Ile
                245                 250                 255

atg gaa aag tct aca atg ttg tat aac aag ttt aag aac atg ttc ttg 816
Met Glu Lys Ser Thr Met Leu Tyr Asn Lys Phe Lys Asn Met Phe Leu
            260                 265                 270

gtt ggt gaa gga gat tcc gtg atc acc caa gtg ctg aat aaa tct ctt 864
Val Gly Glu Gly Asp Ser Val Ile Thr Gln Val Leu Asn Lys Ser Leu
        275                 280                 285

gct gaa caa aga cag cat gag gaa gcg aat aaa acc aaa gat caa ggg 912
Ala Glu Gln Arg Gln His Glu Glu Ala Asn Lys Thr Lys Asp Gln Gly
    290                 295                 300

aag aaa ggg cca aac aaa aag cta gag aag gaa caa aca ggg tca aag 960

Lys Lys Gly Pro Asn Lys Lys Leu Glu Lys Glu Gln Thr Gly Ser Lys
305                 310                 315                 320

act cta aat gga gga tct gat gct caa gat ggt aat cag cca caa cat     1008
Thr Leu Asn Gly Gly Ser Asp Ala Gln Asp Gly Asn Gln Pro Gln His
                325                 330                 335

aac ggg gag agc aat gaa gac agc aaa gac aac cat gaa gcc agc acg     1056
Asn Gly Glu Ser Asn Glu Asp Ser Lys Asp Asn His Glu Ala Ser Thr
            340                 345                 350

aag aaa aag cca tcc agt gaa gag aga gag act gaa ata tct ctg aag     1104
Lys Lys Lys Pro Ser Ser Glu Glu Arg Glu Thr Glu Ile Ser Leu Lys
        355                 360                 365

gat tct aca cta gat aac taggttgaca tacctggaat atagagaaca            1152
Asp Ser Thr Leu Asp Asn
    370

cttgagaagt ttgtaatggt tttcatttga aatagactgc tggaagttta aatttttata 1212

agcataggtt tgatgttgaa aacttgtttt gagggagaaa atccctttgt tttaaagtaa 1272

agtaaacatt atcgctaagt gtaacttgtg cagtattaac agctacatta tacagtaaat 1332

gtgggataaa atccatttag aaaatgttaa actgcttttc cagacatggt tgtagcatat 1392

tttcaattag tgtgtgtatg ttaatgtgta attgatagta gaacaaagtt acatttttaa 1452

aactgctact tgtataaacc ttgcctcttt tcccaaatac tgtgggtttt gtgcatagtt 1512

tttacaaacc ttggatttac cagactgtct tttcactgtt tgtgggtttt gtagaagtta 1572

cacattttta tggtagataa aatgttactt ctatacaagt actcactccc tttttatcaa 1632

aagttaattt taatctcaca gtctacattg tgctacatta tccagcttct ttggaacaat 1692

gtgtgctctg tatggttttt tttggtatga caactaatta agcaactgac attgaactga 1752

gaattctaca aactataaaa cattaatttt tgaaggtaat ttagttttgt ggctgggcat 1812

tcagtgaagt cttaggactt ctttgcagac aactgactgg gtatatatag gaatgaatct 1872

ggctttaggg ttaaatcatt taaggtcctt ttataggcag gcactagtaa ctaaaactga 1932

aaactaagta agtttatttt tgaggaatgt tgttaaaaat gtctttagga agtcactaaa 1992

acttaattgg aagaaaaaat catgatgctt atacaataaa tatgaataaa tgttatataa 2052

ggaaactcac ctatttgaaa tcatggctat attgttttta ttttctagat tccaaaaata 2112

caaacactag ttgttccagc attgtacttt gataagtctg tacattgacg tgtatggact 2172

aaatccaggg taaaatcaat gttacaaaat ttaagggtat gttaactaaa ggatagcatt 2232

tctaagatat tttgaatatt agggtcattt ggcacttctc agcaagtagg atacttctca 2292

tgtttttgaa attatatgaa tatggaaaaa aatggcttaa gaccagcgtc tctgtatgac 2352

attgtgtggt tgaccctctg agataactgt tttcatctac agaattgcat ttttgctttt 2412

aaagaggtct tataatggaa ctaggaatca ccgttttgag agaacctgca tatataccag 2472

tcattatctg tttggtcctt atacagtttt aacttactta gatttattct agttaagcca 2532

taagttcaac gtgtaaactt gttttcatta aagaa 2567

<210> 7
<211> 1173
<212> DNA
<213> Homo sapiens

<220>
<221> CDS
<222> (1)..(543)

<400> 7
ccc cca cca tcc caa caa gtc aac tac att gct tca caa cca gat gga 48
Pro Pro Pro Ser Gln Gln Val Asn Tyr Ile Ala Ser Gln Pro Asp Gly
1               5                   10                  15

aag caa ttg cag ggt att cct agt tct tct cat gta agt aat aac atg 96
Lys Gln Leu Gln Gly Ile Pro Ser Ser Ser His Val Ser Asn Asn Met

            20                  25                  30

agt aca cca gtt ttg cct gct ccg aca gca gcc cca gga aat acg gga 144
Ser Thr Pro Val Leu Pro Ala Pro Thr Ala Ala Pro Gly Asn Thr Gly
        35                  40                  45

atg gtt cag gga cca agt tct ggt aat act tcg tca tca agt cac agc 192
Met Val Gln Gly Pro Ser Ser Gly Asn Thr Ser Ser Ser Ser His Ser
    50                  55                  60

aaa gcc tct aat gct gct gta aaa ttg gca gaa agc aaa gta agt gtt 240
Lys Ala Ser Asn Ala Ala Val Lys Leu Ala Glu Ser Lys Val Ser Val
65                  70                  75                  80

gca gtg gaa gcc agc gca gat agc tcg aag aca gac aag aaa ttg caa 288
Ala Val Glu Ala Ser Ala Asp Ser Ser Lys Thr Asp Lys Lys Leu Gln
                85                  90                  95

att caa gaa aaa gca gca caa gag gta aaa ttg gcc atc aag cca ttt 336
Ile Gln Glu Lys Ala Ala Gln Glu Val Lys Leu Ala Ile Lys Pro Phe
            100                 105                 110

tac caa aat aaa gat atc acc aag gaa gaa tat aaa gaa att gta cgg 384
Tyr Gln Asn Lys Asp Ile Thr Lys Glu Glu Tyr Lys Glu Ile Val Arg
        115                 120                 125

aaa gca gta gat aaa gtt tgt cat agt aag agt gga gaa gta aat tct 432
Lys Ala Val Asp Lys Val Cys His Ser Lys Ser Gly Glu Val Asn Ser
    130                 135                 140

act aaa gtg gca aat ctg gtt aaa gcc tat gta gac aaa tac aaa tat 480
Thr Lys Val Ala Asn Leu Val Lys Ala Tyr Val Asp Lys Tyr Lys Tyr
145                 150                 155                 160

tca cgg aag ggg agc caa aag aaa act ctg gaa gaa cct gtg tct act 528
Ser Arg Lys Gly Ser Gln Lys Lys Thr Leu Glu Glu Pro Val Ser Thr
                165                 170                 175

gaa aaa aac ata ggc tgaaatgggg aacgctgtca aggacattat caggatatct 583
Glu Lys Asn Ile Gly
            180

gcaaagtgca atttcaacat gtaccattaa ctgaaaatca tacataactg tgattgaaat 643

ttggttttga taaaattatt tttttaacat aggatatgat gttttgttct aaataaatat 703

aggtctgcac tgcaacttct gtatccttcc ttcccctcca ccctccccca caaaattcaa 763

gggaaagtaa agggtttaaa ggaatgtgca tctttactag gactgtgtta tagtgtggat 823

actggaaaat gtatagcttt ttgattaggg caatggagtg cataaattag aaactttcta 883

agtgcactgg ttttcaaaga gatatatata atgcatttat tctgtcaggt taaaatataa 943

agtatgatct ttatgatttt ttccctctaa ttatagaaag ttaaataatg tattaccatg 1003

aaaaatgttt ctaatattaa atagaacata tcagttgcaa agttcctaat gtgtattttt 1063

aaagcacata tctgaataaa ttgcctagat agaaaaaaaa ttatcatcga gtaaaattta 1123

gtgttcaaaa cattgagaca ctcttcacct attgtatgac caaataaagg 1173

<210> 8
<211> 2131
<212> DNA
<213> Homo sapiens

<220>
<221> CDS
<222> (1034)..(2131)

<400> 8
tgcggccgca cctgcaggcg cagagtaggt atggaagatc cctcgagatc cattgtgctc 60

taaagccgcc gggggtccgt gtcctgtctc ggttggccgg acccgggccc gagcccgagc 120

agtagccggc gccatgtcgg tggtgggcat agacctgggc ttccagagct gctacgtcgc 180

tgtggcccgc gccggcggca tcgagactat cgctaatgag tatagcgacc gctgcacgcc 240

ggcttgcatt tcttttggtc ctaagaatcg ttcaattgga gcagcagcta aaagccaggt 300

aatttctaat gcaaagaaca cagtccaagg atttaaaaga ttccatggcc gagcattctc 360

tgatccattt gtggaggcag aaaaatctaa ccttgcatat gatattgtgc agtggcctac 420

aggattaaca ggtataaagg tgacatatat ggaggaagag cgaaatttta ccactgagca 480

agtgactgcc atgcttttgt ccaaactgaa ggagacagcc gaaagtgttc ttaagaagcc 540

tgtagttgac tgtgttgttt cggttccttg tttctatact gatgcagaaa gacgatcagt 600

gatggatgca acacagattg ctggtcttaa ttgcttgcga ttaatgaatg aaaccactgc 660

agttgctctt gcatatggaa tctataagca ggatcttcct cgcttagaag agaaaccaag 720

aaatgtagtt tttgtagaca tgggccactc tgcttatcaa gtttctgtat gtgcatttaa 780

tagaggaaaa ctgaaagttc tggccactgc atttgacacg acattgggag gtagaaaatt 840

tgatgaagtg ttagtaaatc acttctgtga agaatttggg aagaaataca agctagacat 900

taagtccaaa atccgtgcat tattacgact ctctcaggag tgtgagaaac tcaagaaatt 960

gatgagtgca aatgcttcag atctcccttt gagcattgaa tgttttatga atgatgttga 1020

tgtatctgga act atg aat aga ggc aaa ttt ctg gag atg tgc aat gat 1069
Met Asn Arg Gly Lys Phe Leu Glu Met Cys Asn Asp
1               5                   10

ctc tta gct aga gtg gag cca cca ctt cgt agt gtt ttg gaa caa acc 1117
Leu Leu Ala Arg Val Glu Pro Pro Leu Arg Ser Val Leu Glu Gln Thr
        15                  20                  25

aag tta aag aaa gaa gat att tat gca gtg gag ata gtt ggt ggt gct 1165
Lys Leu Lys Lys Glu Asp Ile Tyr Ala Val Glu Ile Val Gly Gly Ala
    30                  35                  40

aca cga atc cct gcg gta aaa gag aag atc agc aaa ttt ttc ggt aaa 1213
Thr Arg Ile Pro Ala Val Lys Glu Lys Ile Ser Lys Phe Phe Gly Lys
45                  50                  55                  60

gaa ctt agt aca aca tta aat gct gat gaa gct gtc act cga ggc tgt 1261
Glu Leu Ser Thr Thr Leu Asn Ala Asp Glu Ala Val Thr Arg Gly Cys
                65                  70                  75

gca ttg cag tgt gcc atc tta tcg cct gct ttc aaa gtc aga gaa ttt 1309
Ala Leu Gln Cys Ala Ile Leu Ser Pro Ala Phe Lys Val Arg Glu Phe
            80                  85                  90

tct atc act gat gta gta cca tat cca ata tct ctg aga tgg aat tct 1357
Ser Ile Thr Asp Val Val Pro Tyr Pro Ile Ser Leu Arg Trp Asn Ser
        95                  100                 105

cca gct gaa gaa ggg tca agt gac tgt gaa gtc ttt tcc aaa aat cat 1405
Pro Ala Glu Glu Gly Ser Ser Asp Cys Glu Val Phe Ser Lys Asn His
    110                 115                 120

gct gct cct ttc tct aaa gtt ctt aca ttt tat aga aag gaa cct ttc 1453
Ala Ala Pro Phe Ser Lys Val Leu Thr Phe Tyr Arg Lys Glu Pro Phe
125                 130                 135                 140

act ctt gag gcc tac tac agc tct cct cag gat ttg ccc tat cca gat 1501
Thr Leu Glu Ala Tyr Tyr Ser Ser Pro Gln Asp Leu Pro Tyr Pro Asp
                145                 150                 155

cct gct ata gct cag ttt tca gtt cag aaa gtc act cct cag tct gat 1549
Pro Ala Ile Ala Gln Phe Ser Val Gln Lys Val Thr Pro Gln Ser Asp
            160                 165                 170

ggc tcc agt tca aaa gtg aaa gtc aaa gtt cga gta aat gtc cat ggc 1597
Gly Ser Ser Ser Lys Val Lys Val Lys Val Arg Val Asn Val His Gly
        175                 180                 185

att ttc agt gtg tcc agt gca tct tta gtg gag gtt cac aag tct gag 1645
Ile Phe Ser Val Ser Ser Ala Ser Leu Val Glu Val His Lys Ser Glu
    190                 195                 200

gaa aat gag gag cca atg gaa aca gat cag aat gca aag gag gaa gag 1693
Glu Asn Glu Glu Pro Met Glu Thr Asp Gln Asn Ala Lys Glu Glu Glu
205                 210                 215                 220

aag atg caa gtg gac cag gag gaa cca cat gtt gaa gag caa cag cag 1741
Lys Met Gln Val Asp Gln Glu Glu Pro His Val Glu Glu Gln Gln Gln
                225                 230                 235

cag aca cca gca gaa aat aag gca gag tct gaa gaa atg gag acc tct 1789

Gln Thr Pro Ala Glu Asn Lys Ala Glu Ser Glu Glu Met Glu Thr Ser
            240                 245                 250

caa gct gga tcc aag gat aaa aag atg gac caa cca ccc caa tgc caa 1837
Gln Ala Gly Ser Lys Asp Lys Lys Met Asp Gln Pro Pro Gln Cys Gln
        255                 260                 265

gaa ggc aaa agt gaa gac cag tac tgt gga cct gcc aat cga gaa tca 1885
Glu Gly Lys Ser Glu Asp Gln Tyr Cys Gly Pro Ala Asn Arg Glu Ser
    270                 275                 280

gct ata tgg cag ata gac aga gag atg ctc aac ttg tac att gaa aat 1933
Ala Ile Trp Gln Ile Asp Arg Glu Met Leu Asn Leu Tyr Ile Glu Asn
285                 290                 295                 300

gag ggt aag atg atc atg cag gat aaa ctg gag aag gag cgg aat gat 1981
Glu Gly Lys Met Ile Met Gln Asp Lys Leu Glu Lys Glu Arg Asn Asp
                305                 310                 315

gct aag aac gca gtg gag gaa tat gtg tat gaa atg aga gac aag ctt 2029
Ala Lys Asn Ala Val Glu Glu Tyr Val Tyr Glu Met Arg Asp Lys Leu
            320                 325                 330

agt ggt gaa tat gag aag ttt gtg agt gaa gat gat cgt aac agt ttt 2077
Ser Gly Glu Tyr Glu Lys Phe Val Ser Glu Asp Asp Arg Asn Ser Phe
        335                 340                 345

act ttg aaa ctg gaa gat act gaa aat tgg ttg tat gag gat gga gaa 2125
Thr Leu Lys Leu Glu Asp Thr Glu Asn Trp Leu Tyr Glu Asp Gly Glu
    350                 355                 360

gac cag 2131
Asp Gln
365

<210> 9
<211> 2106
<212> DNA
<213> Homo sapiens

<220>

<221> CDS
<222> (1)..(1788)

<400> 9
gga tca gaa aag ttt ctg act ctc atg aag atg cca gtc atc tct ttt     48
Gly Ser Glu Lys Phe Leu Thr Leu Met Lys Met Pro Val Ile Ser Phe
1               5                   10                  15

gtg cgt cta gga tac tgt agc ttc tct aag tcc aga atc ctc aac aca     96
Val Arg Leu Gly Tyr Cys Ser Phe Ser Lys Ser Arg Ile Leu Asn Thr
            20                  25                  30

ctt ctc agc cct gcc cag ttg aaa tta cac aaa atc ttt ctt cat caa     144
Leu Leu Ser Pro Ala Gln Leu Lys Leu His Lys Ile Phe Leu His Gln
        35                  40                  45

gat ttg cct ctt ttg gtg ctt ccc cgg caa atc tct gat ggc ctg gtt     192
Asp Leu Pro Leu Leu Val Leu Pro Arg Gln Ile Ser Asp Gly Leu Val
    50                  55                  60

gag ata aca tgg tgt ttt cct gat agc gat gat aga aag gaa aac ccc     240
Glu Ile Thr Trp Cys Phe Pro Asp Ser Asp Asp Arg Lys Glu Asn Pro
65                  70                  75                  80

ttt ttc caa aag cct gtt gct ctg gct aat ctc cgt gga aat cta gaa     288
Phe Phe Gln Lys Pro Val Ala Leu Ala Asn Leu Arg Gly Asn Leu Glu
                85                  90                  95

agc ttt tgg act cag ttt ggt ttt ttg atg gaa gtt tct tca gct gtg     336
Ser Phe Trp Thr Gln Phe Gly Phe Leu Met Glu Val Ser Ser Ala Val
            100                 105                 110

ttt ttt ttc act gac tgt tta ggt gag aag gaa tgg gac ttg cta atg     384
Phe Phe Phe Thr Asp Cys Leu Gly Glu Lys Glu Trp Asp Leu Leu Met
        115                 120                 125

ttt tta gga gag gct gcc att gaa aga tgc tac ttt gtt ctc agt tcc     432
Phe Leu Gly Glu Ala Ala Ile Glu Arg Cys Tyr Phe Val Leu Ser Ser
    130                 135                 140

caa gcc agg gag agt gaa gag gct caa att ttt cag agg ata ctg aac     480
Gln Ala Arg Glu Ser Glu Glu Ala Gln Ile Phe Gln Arg Ile Leu Asn

145                 150                 155                 160

ttg aag cca gca cag cta ctg ttt tgg gag agg gga gat gct ggg gat     528
Leu Lys Pro Ala Gln Leu Leu Phe Trp Glu Arg Gly Asp Ala Gly Asp
                165                 170                 175

aga agg aag aac atg gag ggc ctt caa gct gcc ctc cag gaa gtg atg     576
Arg Arg Lys Asn Met Glu Gly Leu Gln Ala Ala Leu Gln Glu Val Met
            180                 185                 190

ttc tct tct tgc ctc aga tgt gtg tct gtg gag gat atg gcc gcc ctg     624
Phe Ser Ser Cys Leu Arg Cys Val Ser Val Glu Asp Met Ala Ala Leu
        195                 200                 205

gcc agg gag ctg ggg att cag gta gat gaa gac ttt gaa aac act cag     672
Ala Arg Glu Leu Gly Ile Gln Val Asp Glu Asp Phe Glu Asn Thr Gln
    210                 215                 220

aga att caa gtt tcc tct gga gaa aac atg gct ggg aca gct gaa ggt     720
Arg Ile Gln Val Ser Ser Gly Glu Asn Met Ala Gly Thr Ala Glu Gly
225                 230                 235                 240

gag ggt cag caa aga cac agt cag cta aaa agc tca tct aaa agc cag     768
Glu Gly Gln Gln Arg His Ser Gln Leu Lys Ser Ser Ser Lys Ser Gln
                245                 250                 255

gct cta atg cca att caa gag cct ggg act caa tgt gag ctc agc cag     816
Ala Leu Met Pro Ile Gln Glu Pro Gly Thr Gln Cys Glu Leu Ser Gln
            260                 265                 270

aat ctt cag aat ctc tat ggt acc cca gta ttc agg cct gtt cta gag     864
Asn Leu Gln Asn Leu Tyr Gly Thr Pro Val Phe Arg Pro Val Leu Glu
        275                 280                 285

aac tcc tgg ctc ttt cca acc aga att gga ggt aac ttt aac cat gtt     912
Asn Ser Trp Leu Phe Pro Thr Arg Ile Gly Gly Asn Phe Asn His Val
    290                 295                 300

tcc ttg aaa gcc tcc tgg gtt atg ggc cgc ccc ttt ggg tca gag cag     960
Ser Leu Lys Ala Ser Trp Val Met Gly Arg Pro Phe Gly Ser Glu Gln
305                 310                 315                 320

agg cct aag tgg ttc cat cct ttg cct ttt cag aat gca ggg gcc cag 1008
Arg Pro Lys Trp Phe His Pro Leu Pro Phe Gln Asn Ala Gly Ala Gln
                325                 330                 335

ggc cga ggt aaa agt ttt ggt att caa tcc ttc cat ccc cag ata ttt 1056
Gly Arg Gly Lys Ser Phe Gly Ile Gln Ser Phe His Pro Gln Ile Phe
            340                 345                 350

tat tca ggt gaa aga ttc atg aaa ttt tcc aga gtt gct cgg gga tgt 1104
Tyr Ser Gly Glu Arg Phe Met Lys Phe Ser Arg Val Ala Arg Gly Cys
        355                 360                 365

cac tcg aat gga aca ttt ggg aga ctg cca aga ccc att tgt cag cat 1152
His Ser Asn Gly Thr Phe Gly Arg Leu Pro Arg Pro Ile Cys Gln His
    370                 375                 380

gta cag gcc tgc cct gag aga cca caa atg atg gga act ctt gaa agg 1200
Val Gln Ala Cys Pro Glu Arg Pro Gln Met Met Gly Thr Leu Glu Arg
385                 390                 395                 400

tct agg gca gta gcc tcc aag ata ggt cac tcc tat tcc ctg gat tca 1248
Ser Arg Ala Val Ala Ser Lys Ile Gly His Ser Tyr Ser Leu Asp Ser
                405                 410                 415

cag cca gca aga gca gta ggg aag cca tgg cct cag caa gct tgc acc 1296
Gln Pro Ala Arg Ala Val Gly Lys Pro Trp Pro Gln Gln Ala Cys Thr
            420                 425                 430

agg gta aca gag tta act gaa gca act gga aaa ctg ata aga aca tcc 1344
Arg Val Thr Glu Leu Thr Glu Ala Thr Gly Lys Leu Ile Arg Thr Ser
        435                 440                 445

cat att gga aag cct cac cct cag tcc ttt caa cca gca gca gcc aca 1392
His Ile Gly Lys Pro His Pro Gln Ser Phe Gln Pro Ala Ala Ala Thr
    450                 455                 460

caa aaa cta aga cct gct tct cag caa gga gtc cag atg aag aca caa 1440
Gln Lys Leu Arg Pro Ala Ser Gln Gln Gly Val Gln Met Lys Thr Gln
465                 470                 475                 480

ggt ggg gct tca aat cca gct ctc caa ata ggg tcc cat ccc atg tgc 1488
Gly Gly Ala Ser Asn Pro Ala Leu Gln Ile Gly Ser His Pro Met Cys

                485                 490                 495

aag agc tct cag ttc aaa tcc gat cag tcc aac cca tcc aca gtc aaa 1536
Lys Ser Ser Gln Phe Lys Ser Asp Gln Ser Asn Pro Ser Thr Val Lys
            500                 505                 510

cac tcc cag cct aaa ccc ttc cat tct gtg ccc tct caa cct aaa tcc 1584
His Ser Gln Pro Lys Pro Phe His Ser Val Pro Ser Gln Pro Lys Ser
        515                 520                 525

tct cag aca aaa tcc tgt cag tcc cag ccc tcc caa act aaa cct tct 1632
Ser Gln Thr Lys Ser Cys Gln Ser Gln Pro Ser Gln Thr Lys Pro Ser
    530                 535                 540

cca tgc aaa tct act cag cct aag cca agc cag ccc tgg cct ccc cag 1680
Pro Cys Lys Ser Thr Gln Pro Lys Pro Ser Gln Pro Trp Pro Pro Gln
545                 550                 555                 560

tct aag cct tct cag ccc aga ccc cct caa cct aag tca tcc tca acc 1728
Ser Lys Pro Ser Gln Pro Arg Pro Pro Gln Pro Lys Ser Ser Ser Thr
                565                 570                 575

aat cct tca caa gct aag gca cac cac tca aaa gca ggg cag aag agg 1776
Asn Pro Ser Gln Ala Lys Ala His His Ser Lys Ala Gly Gln Lys Arg
            580                 585                 590

gga ggg aag cat taaagagcta actccagaga tctataaagc atatccttta 1828
Gly Gly Lys His
        595

cccaggccat tcctatcata tagtaagcag aagagttgcc atgaaagtaa aagactactg 1888

tcattagcat gtaaaacaaa gaaagatata catgaccgaa ttggatatct ttgtttgttt 1948

gtttgagaca gagttcactc ttgtgcccag gctggagtgc aatggcacga tctcggctca 2008

ccgaacctct gcttcctggc ttaaagtgat tctcctgctc agcctctcga gtagctggga 2068

ttacaggcat tgcaccacca cacccagtta ttttgttt 2106

<210> 10

<211> 7450
<212> DNA
<213> Homo sapiens

<220>
<221> CDS
<222> (277)..(6921)

<400> 10
cccccctttt ttttttttaa ttcttcagct aaaacagcgg aagaggtgat ttattatatg 60

gttgttacac tcggccacaa ataaacacag aaatagtcca gaatgtcaca ggtccagggc 120

agaggaccaa catgggcatt ttgtttatga gcaaggtggg tctcagaggt gatcggcgat 180

cagagggcga tgaagttcta gatccattga gacaagctct agacagtagc atgcagtccc 240

acaacttgta ccagcatccc cagcgtctgg cattcc atg ttt ctg ctc ctg tgg 294
Met Phe Leu Leu Leu Trp
1               5

cct cca cgg tgc aac aag cta gcg gtt tac ttg gac ctc tgc ctc atc 342
Pro Pro Arg Cys Asn Lys Leu Ala Val Tyr Leu Asp Leu Cys Leu Ile
            10                  15                  20

ttt ctt ctt ttg cgc ttc agc ctg cgc att cgc ttc ttc ctc cac ttg 390
Phe Leu Leu Leu Arg Phe Ser Leu Arg Ile Arg Phe Phe Leu His Leu
        25                  30                  35

gct ctc atg gcg cag agg ttt cca gag agc ctg tca caa aag cat cct 438
Ala Leu Met Ala Gln Arg Phe Pro Glu Ser Leu Ser Gln Lys His Pro
    40                  45                  50

cag cac tct gtg gag tat gag ggt aac att cat aca agt tta gcc att 486
Gln His Ser Val Glu Tyr Glu Gly Asn Ile His Thr Ser Leu Ala Ile
55                  60                  65                  70

gct caa aag cta atg gaa ccg aaa ttg ggg aaa ata aat caa aat tat 534
Ala Gln Lys Leu Met Glu Pro Lys Leu Gly Lys Ile Asn Gln Asn Tyr
                75                  80                  85

gct agc att ata act gaa gct ttc ccg aaa cca aaa gac ata ccc cag 582

Ala Ser Ile Ile Thr Glu Ala Phe Pro Lys Pro Lys Asp Ile Pro Gln
            90                  95                  100

gcc aaa gaa atg ttc att gat aca gtt att tca tct tat aac ata gaa 630
Ala Lys Glu Met Phe Ile Asp Thr Val Ile Ser Ser Tyr Asn Ile Glu
        105                 110                 115

aca gct cat gac agt tca aat tgc agc ata act aga gaa cat ata tgt 678
Thr Ala His Asp Ser Ser Asn Cys Ser Ile Thr Arg Glu His Ile Cys
    120                 125                 130

gtc cat agg aaa aat gaa aat gaa cca gtg tca tta gag acc att cag 726
Val His Arg Lys Asn Glu Asn Glu Pro Val Ser Leu Glu Thr Ile Gln
135                 140                 145                 150

aga gac tat aaa gaa act gct tat gtt gaa gat agg ggt cag gat cac 774
Arg Asp Tyr Lys Glu Thr Ala Tyr Val Glu Asp Arg Gly Gln Asp His
                155                 160                 165

aat ctg ttc tgt aat tca cag tta agc aat gat ata tgg ctg aat gtt 822
Asn Leu Phe Cys Asn Ser Gln Leu Ser Asn Asp Ile Trp Leu Asn Val
            170                 175                 180

aat ttc aaa aaa caa aca gat aga gaa aac caa aat gag gct aaa gag 870
Asn Phe Lys Lys Gln Thr Asp Arg Glu Asn Gln Asn Glu Ala Lys Glu
        185                 190                 195

aat agt gct tca tgt gta gaa aac aac ata gag aac ata tat gga gac 918
Asn Ser Ala Ser Cys Val Glu Asn Asn Ile Glu Asn Ile Tyr Gly Asp
    200                 205                 210

aaa aag cag gat tct cat aca aac gaa aat ttc agc aat ata gat gaa 966
Lys Lys Gln Asp Ser His Thr Asn Glu Asn Phe Ser Asn Ile Asp Glu
215                 220                 225                 230

aag gag gac aaa aat tac cac aat ata gaa att ttg agt tct gaa gaa 1014
Lys Glu Asp Lys Asn Tyr His Asn Ile Glu Ile Leu Ser Ser Glu Glu
                235                 240                 245

ttt tct act aaa ttt aac ttg att tgc aga gaa gat aat gca gtg tca 1062
Phe Ser Thr Lys Phe Asn Leu Ile Cys Arg Glu Asp Asn Ala Val Ser
            250                 255                 260

gca gca act gca tta tta gag agt gaa gaa gat acc att agt gcc gtg 1110
Ala Ala Thr Ala Leu Leu Glu Ser Glu Glu Asp Thr Ile Ser Ala Val
        265                 270                 275

aaa caa aaa gat act gaa aat act gga aga agt gta gag cat ttg gct 1158
Lys Gln Lys Asp Thr Glu Asn Thr Gly Arg Ser Val Glu His Leu Ala
    280                 285                 290

tcc acg aca ttt ccc aaa act gca agt tct tca gtg tgt gta gcc tca 1206
Ser Thr Thr Phe Pro Lys Thr Ala Ser Ser Ser Val Cys Val Ala Ser
295                 300                 305                 310

aat gct gca ata cag ata gct agt gct act atg cct gca tta agc cta 1254
Asn Ala Ala Ile Gln Ile Ala Ser Ala Thr Met Pro Ala Leu Ser Leu
                315                 320                 325

aat aat gac gat cac cag ata tac cag ttt aaa gaa act tgt tct tct 1302
Asn Asn Asp Asp His Gln Ile Tyr Gln Phe Lys Glu Thr Cys Ser Ser
            330                 335                 340

gaa agt cca gat ttt ggt ttg tta gta aaa cat agg gtt tct gat tgt 1350
Glu Ser Pro Asp Phe Gly Leu Leu Val Lys His Arg Val Ser Asp Cys
        345                 350                 355

gaa att gat acg gat aaa aat aaa tca caa gaa tca ttt cat caa tca 1398
Glu Ile Asp Thr Asp Lys Asn Lys Ser Gln Glu Ser Phe His Gln Ser
    360                 365                 370

ata aat gag aac tta gtt ctt cag agc att gaa ttg gaa agt gaa att 1446
Ile Asn Glu Asn Leu Val Leu Gln Ser Ile Glu Leu Glu Ser Glu Ile
375                 380                 385                 390

gaa ata gaa tta gaa gat tgt gat gat gct ttt ata ttt caa caa gat 1494
Glu Ile Glu Leu Glu Asp Cys Asp Asp Ala Phe Ile Phe Gln Gln Asp
                395                 400                 405

aca cat agc cat gaa aac atg ctt tgt gaa gaa ttt gtg acc tca tat 1542
Thr His Ser His Glu Asn Met Leu Cys Glu Glu Phe Val Thr Ser Tyr
            410                 415                 420

aag gct ctg aag tct cgt atc agt tgg gaa ggt ctg tta gca ctt gat 1590

Lys Ala Leu Lys Ser Arg Ile Ser Trp Glu Gly Leu Leu Ala Leu Asp
        425                 430                 435

aac ggg gag atg gaa gtt ttg gaa agc acc aca gga agg gag aat agt    1638
Asn Gly Glu Met Glu Val Leu Glu Ser Thr Thr Gly Arg Glu Asn Ser
    440                 445                 450

gat cag cat tat tct aag gaa agt aac tat ttt tat tcc tct aca caa    1686
Asp Gln His Tyr Ser Lys Glu Ser Asn Tyr Phe Tyr Ser Ser Thr Gln
455                 460                 465                 470

aac aat gaa aca gaa ctt acc agc cca att tta ctt cca gat cta caa    1734
Asn Asn Glu Thr Glu Leu Thr Ser Pro Ile Leu Leu Pro Asp Leu Gln
                475                 480                 485

att aaa att act aat ata ttt agg cca gga ttc agc ccg aca gct gac    1782
Ile Lys Ile Thr Asn Ile Phe Arg Pro Gly Phe Ser Pro Thr Ala Asp
            490                 495                 500

tcc ctt gca ttg aaa gat agt ttt tgc aca cat gta act gaa gcc aca    1830
Ser Leu Ala Leu Lys Asp Ser Phe Cys Thr His Val Thr Glu Ala Thr
        505                 510                 515

aaa ccg gaa ata aat aag gaa gat gga gaa att cta gga ttt gac att    1878
Lys Pro Glu Ile Asn Lys Glu Asp Gly Glu Ile Leu Gly Phe Asp Ile
    520                 525                 530

tat tcc cag cct ttt ggt gaa aat gca gat tat cca tgt gaa gat aaa    1926
Tyr Ser Gln Pro Phe Gly Glu Asn Ala Asp Tyr Pro Cys Glu Asp Lys
535                 540                 545                 550

gtt gat aat ata agg caa gaa tca ggg cca gtg agt aac tct gaa atc    1974
Val Asp Asn Ile Arg Gln Glu Ser Gly Pro Val Ser Asn Ser Glu Ile
                555                 560                 565

tcc ctt tct ttt gac ttg agt cgt aat aca gat gtg aat cat acg tct    2022
Ser Leu Ser Phe Asp Leu Ser Arg Asn Thr Asp Val Asn His Thr Ser
            570                 575                 580

gaa aat cag aac agt gaa tct ttg ttt act gaa cct tct aat gtc aca    2070
Glu Asn Gln Asn Ser Glu Ser Leu Phe Thr Glu Pro Ser Asn Val Thr
        585                 590                 595

aca ata gat gat gga agc aga tgt ttc ttt aca aaa tca aaa act gac 2118
Thr Ile Asp Asp Gly Ser Arg Cys Phe Phe Thr Lys Ser Lys Thr Asp
    600                 605                 610

tat aat gat acc aaa aat aaa aag gag gta gaa tca aga att agc aaa 2166
Tyr Asn Asp Thr Lys Asn Lys Lys Glu Val Glu Ser Arg Ile Ser Lys
615                 620                 625                 630

agg aag cta cat ata tct tcc agg gat cag aac ata cca cat aaa gat 2214
Arg Lys Leu His Ile Ser Ser Arg Asp Gln Asn Ile Pro His Lys Asp
                635                 640                 645

tta aga cga cat aaa att tat ggg aga aag agg agg cta acc agt caa 2262
Leu Arg Arg His Lys Ile Tyr Gly Arg Lys Arg Arg Leu Thr Ser Gln
            650                 655                 660

gac tca tct gag tgt ttc tct tca tta tcc caa gga cga att aaa aca 2310
Asp Ser Ser Glu Cys Phe Ser Ser Leu Ser Gln Gly Arg Ile Lys Thr
        665                 670                 675

ttt tca cag tca gaa aag cac att aag agt gtc cta aat atc cta agt 2358
Phe Ser Gln Ser Glu Lys His Ile Lys Ser Val Leu Asn Ile Leu Ser
    680                 685                 690

gat gaa gca tct tta tgt aaa agc aaa tgt ctt tcc aga aaa cta gac 2406
Asp Glu Ala Ser Leu Cys Lys Ser Lys Cys Leu Ser Arg Lys Leu Asp
695                 700                 705                 710

aaa gca gtt gtt cac tta aaa aaa gct cat aga aga gtt cac aca tct 2454
Lys Ala Val Val His Leu Lys Lys Ala His Arg Arg Val His Thr Ser
                715                 720                 725

ttg cag ctt ata act aaa gta gga gaa gaa aga aag ggc cca tta cca 2502
Leu Gln Leu Ile Thr Lys Val Gly Glu Glu Arg Lys Gly Pro Leu Pro
            730                 735                 740

aaa tca tat gca ata ata tgc aat aat ttc tgg gaa agt tgt gac ctt 2550
Lys Ser Tyr Ala Ile Ile Cys Asn Asn Phe Trp Glu Ser Cys Asp Leu
        745                 750                 755

caa ggt tat agt tct gtg tct caa aga aaa tat tat tct act aag cat 2598

Gln Gly Tyr Ser Ser Val Ser Gln Arg Lys Tyr Tyr Ser Thr Lys His
    760                 765                 770

ttt tcg tca aaa aga aaa tat gac aaa cgg aga aag aaa aga gct cca 2646
Phe Ser Ser Lys Arg Lys Tyr Asp Lys Arg Arg Lys Lys Arg Ala Pro
775                 780                 785                 790

aaa gct gat att tct aaa tca tta acc cat gtg tca aag cac aag tct 2694
Lys Ala Asp Ile Ser Lys Ser Leu Thr His Val Ser Lys His Lys Ser
                795                 800                 805

tat aaa aca agt gga gag aaa aaa tgc ctt tct agg aaa agt atg gct 2742
Tyr Lys Thr Ser Gly Glu Lys Lys Cys Leu Ser Arg Lys Ser Met Ala
            810                 815                 820

agc agt gtc tca aaa agt cac ccc acc acc agt cac atg gga gaa ttt 2790
Ser Ser Val Ser Lys Ser His Pro Thr Thr Ser His Met Gly Glu Phe
        825                 830                 835

tgt aat caa gaa cat cct gaa tca cag ttg cct gta tcc tcc aca tcc 2838
Cys Asn Gln Glu His Pro Glu Ser Gln Leu Pro Val Ser Ser Thr Ser
    840                 845                 850

caa agt aca agt cag tca gtt tat tat aat agc agt gta agc aat cca 2886
Gln Ser Thr Ser Gln Ser Val Tyr Tyr Asn Ser Ser Val Ser Asn Pro
855                 860                 865                 870

agt tta tca gaa gaa cat cag ccc ttt tct gga aaa act gca tat ctg 2934
Ser Leu Ser Glu Glu His Gln Pro Phe Ser Gly Lys Thr Ala Tyr Leu
                875                 880                 885

ttt tcc cca gac cac tca gat gag aaa cta ata gaa aaa gaa aat caa 2982
Phe Ser Pro Asp His Ser Asp Glu Lys Leu Ile Glu Lys Glu Asn Gln
            890                 895                 900

att gat aca gca ttt tta tct agc act agt aaa tat gaa aag ctt gaa 3030
Ile Asp Thr Ala Phe Leu Ser Ser Thr Ser Lys Tyr Glu Lys Leu Glu
        905                 910                 915

aaa cat tca gca aat cat aat gtt aaa gat gca act aaa gaa aac agt 3078
Lys His Ser Ala Asn His Asn Val Lys Asp Ala Thr Lys Glu Asn Ser
    920                 925                 930

tgt gac gct aat gaa gta ata aat gaa agt aat tct gta tct tta agt 3126
Cys Asp Ala Asn Glu Val Ile Asn Glu Ser Asn Ser Val Ser Leu Ser
935                 940                 945                 950

tgc ata aaa gaa aac ata aat tct agt aca ggc aac gat tgt gat gca 3174
Cys Ile Lys Glu Asn Ile Asn Ser Ser Thr Gly Asn Asp Cys Asp Ala
                955                 960                 965

act tgc ata ggt cac aca aag gcg aaa act gac gta ctt ata tca gtc 3222
Thr Cys Ile Gly His Thr Lys Ala Lys Thr Asp Val Leu Ile Ser Val
            970                 975                 980

tta gat tca aat gtg aag cac ttt tta aat gat ctc tac caa caa ggt 3270
Leu Asp Ser Asn Val Lys His Phe Leu Asn Asp Leu Tyr Gln Gln Gly
        985                 990                 995

aac ctt att tta tct gat tgt aaa aga aac ctg gaa gta aag tgg aca 3318
Asn Leu Ile Leu Ser Asp Cys Lys Arg Asn Leu Glu Val Lys Trp Thr
    1000                1005                1010

gat cct att gag aga ccc ata caa aac att att aca gga aac ttc ctt 3366
Asp Pro Ile Glu Arg Pro Ile Gln Asn Ile Ile Thr Gly Asn Phe Leu
1015                1020                1025                1030

atg ggc cca tta aac cta act ttg ata gca agt aaa aag tac agt att 3414
Met Gly Pro Leu Asn Leu Thr Leu Ile Ala Ser Lys Lys Tyr Ser Ile
                1035                1040                1045

cct cag tta tca gcc gct gca gtg aca gat agt gag gga gaa tct tca 3462
Pro Gln Leu Ser Ala Ala Ala Val Thr Asp Ser Glu Gly Glu Ser Ser
            1050                1055                1060

aaa tct tac ttg gat aag cag aga att ctt act gta gat tct ttt gca 3510
Lys Ser Tyr Leu Asp Lys Gln Arg Ile Leu Thr Val Asp Ser Phe Ala
        1065                1070                1075

gca tcc agt act gta cca cac tgt gag cag agc tgt aga gaa aaa gag 3558
Ala Ser Ser Thr Val Pro His Cys Glu Gln Ser Cys Arg Glu Lys Glu
    1080                1085                1090

ctt cta aag aca gaa cag tgc tct tca ggt aat tgc ctc cat aca gat 3606

Leu Leu Lys Thr Glu Gln Cys Ser Ser Gly Asn Cys Leu His Thr Asp
1095                1100                1105                1110

ggg aat gaa aca aat gtc act gag aat tat gag ttg gat gta gca tca    3654
Gly Asn Glu Thr Asn Val Thr Glu Asn Tyr Glu Leu Asp Val Ala Ser
                1115                1120                1125

gga act gaa gaa gat aaa agt tat ggg gaa aat ata gtg gaa tta tct    3702
Gly Thr Glu Glu Asp Lys Ser Tyr Gly Glu Asn Ile Val Glu Leu Ser
            1130                1135                1140

tcc agt gat agt tct ctg ctt tta aaa gat aat gta aaa ggc tcc tct    3750
Ser Ser Asp Ser Ser Leu Leu Leu Lys Asp Asn Val Lys Gly Ser Ser
        1145                1150                1155

tca gaa aca tgt att gtg aag aaa gac act gag gac aga ata acg tgg    3798
Ser Glu Thr Cys Ile Val Lys Lys Asp Thr Glu Asp Arg Ile Thr Trp
    1160                1165                1170

aaa gtt aaa caa gcg gaa aaa gca aaa gat tct gtt tac aaa aga agc    3846
Lys Val Lys Gln Ala Glu Lys Ala Lys Asp Ser Val Tyr Lys Arg Ser
1175                1180                1185                1190

atg act gaa gga tca act gtt aat act gag tac aaa aat caa aag aat    3894
Met Thr Glu Gly Ser Thr Val Asn Thr Glu Tyr Lys Asn Gln Lys Asn
                1195                1200                1205

cag atc tca gaa gaa tcc tgc tta aat gag aaa att att aca act aac    3942
Gln Ile Ser Glu Glu Ser Cys Leu Asn Glu Lys Ile Ile Thr Thr Asn
            1210                1215                1220

ttg att gat tcc cat ctg agc act aaa aat act acc act gag tca gtc    3990
Leu Ile Asp Ser His Leu Ser Thr Lys Asn Thr Thr Thr Glu Ser Val
        1225                1230                1235

cct ttg aag aac aca gtt tct aat ccg ctt aac aaa aga gag aag aac    4038
Pro Leu Lys Asn Thr Val Ser Asn Pro Leu Asn Lys Arg Glu Lys Asn
    1240                1245                1250

ccc caa att aaa gtt agt aaa gac tcg cag tct gac ttg aca tta cat    4086
Pro Gln Ile Lys Val Ser Lys Asp Ser Gln Ser Asp Leu Thr Leu His
1255                1260                1265                1270

tca gaa ata gcc tat att tcc aaa cca gga att cta gga gtt aat cat 4134
Ser Glu Ile Ala Tyr Ile Ser Lys Pro Gly Ile Leu Gly Val Asn His
                1275                1280                1285

acg cct att tta cct gcc cac tct gaa acc tgt aaa gtc cct act ctt 4182
Thr Pro Ile Leu Pro Ala His Ser Glu Thr Cys Lys Val Pro Thr Leu
            1290                1295                1300

ctg aag aaa cct gcg tca tac gtg agt gat ttt aaa gaa aaa cat tgc 4230
Leu Lys Lys Pro Ala Ser Tyr Val Ser Asp Phe Lys Glu Lys His Cys
        1305                1310                1315

tca gct aat cat acg gcc ctt ata gct aat cta tct caa att ttg cag 4278
Ser Ala Asn His Thr Ala Leu Ile Ala Asn Leu Ser Gln Ile Leu Gln
    1320                1325                1330

agg gca gat gaa gca tca tct ttg cag att cta cag gaa gaa act aag 4326
Arg Ala Asp Glu Ala Ser Ser Leu Gln Ile Leu Gln Glu Glu Thr Lys
1335                1340                1345                1350

gtt tgt cta aat att ctc cct tta ttt gtg gaa gct ttt gaa aga aag 4374
Val Cys Leu Asn Ile Leu Pro Leu Phe Val Glu Ala Phe Glu Arg Lys
                1355                1360                1365

caa gaa tgt tca gtt gaa caa atc ctg att tca aga gaa ctg ttg gta 4422
Gln Glu Cys Ser Val Glu Gln Ile Leu Ile Ser Arg Glu Leu Leu Val
            1370                1375                1380

gac caa aac ctg tgg aat aat tgc aaa cac aca tta aaa cca tgt gct 4470
Asp Gln Asn Leu Trp Asn Asn Cys Lys His Thr Leu Lys Pro Cys Ala
        1385                1390                1395

gtt gac act ttg gta gaa ctt caa atg atg atg gaa aca att caa ttc 4518
Val Asp Thr Leu Val Glu Leu Gln Met Met Met Glu Thr Ile Gln Phe
    1400                1405                1410

att gaa aac aaa aaa agg cac tta gaa ggt gaa cca aca ttg cga agc 4566
Ile Glu Asn Lys Lys Arg His Leu Glu Gly Glu Pro Thr Leu Arg Ser
1415                1420                1425                1430

ttg ctt tgg tat gat gaa aca ctg tat gct gag ctt ctt gga aaa cca 4614

Leu Leu Trp Tyr Asp Glu Thr Leu Tyr Ala Glu Leu Leu Gly Lys Pro
                1435                1440                1445

cgt gga ttt caa cag cag tct aat ttc tat cct ggt ttc caa gga aga 4662
Arg Gly Phe Gln Gln Gln Ser Asn Phe Tyr Pro Gly Phe Gln Gly Arg
            1450                1455                1460

tta aaa tat aat gca ttc tgt gag tta cag act tac cat gat caa tta 4710
Leu Lys Tyr Asn Ala Phe Cys Glu Leu Gln Thr Tyr His Asp Gln Leu
        1465                1470                1475

gtt gaa ttg ctt gag gaa aca aaa agg gaa aag aat tca tac tat gta 4758
Val Glu Leu Leu Glu Glu Thr Lys Arg Glu Lys Asn Ser Tyr Tyr Val
    1480                1485                1490

ttc tta aag tac aaa cga cag gtt aat gaa tgt gaa gcc ata atg gag 4806
Phe Leu Lys Tyr Lys Arg Gln Val Asn Glu Cys Glu Ala Ile Met Glu
1495                1500                1505                1510

cat tgt tcc gat tgc ttt gat ttt tct ctt tct gtt cca ttt acc tgt 4854
His Cys Ser Asp Cys Phe Asp Phe Ser Leu Ser Val Pro Phe Thr Cys
                1515                1520                1525

gga gtt aac ttt gga gat agt tta gaa gac ctg gaa atc tta aga aaa 4902
Gly Val Asn Phe Gly Asp Ser Leu Glu Asp Leu Glu Ile Leu Arg Lys
            1530                1535                1540

agt act tta aag ttg atc aat gta tgt ggg gac tct cct aaa gtt cat 4950
Ser Thr Leu Lys Leu Ile Asn Val Cys Gly Asp Ser Pro Lys Val His
        1545                1550                1555

tcg tat cca gga aaa cag gac cat ctg tgg att atc ata gaa atg atc 4998
Ser Tyr Pro Gly Lys Gln Asp His Leu Trp Ile Ile Ile Glu Met Ile
    1560                1565                1570

tcc tca aag gtt aat ttt att aag aac aac gag gca gta cgt gtt aaa 5046
Ser Ser Lys Val Asn Phe Ile Lys Asn Asn Glu Ala Val Arg Val Lys
1575                1580                1585                1590

ata tct ctt tat ggt ctg gaa cat atc ttt ttt gat gct gca aaa aat 5094
Ile Ser Leu Tyr Gly Leu Glu His Ile Phe Phe Asp Ala Ala Lys Asn
                1595                1600                1605

ctt gtt tgg aaa gag aga aca caa tcc ttc agc aaa aaa tac tca caa 5142
Leu Val Trp Lys Glu Arg Thr Gln Ser Phe Ser Lys Lys Tyr Ser Gln
            1610                1615                1620

aag aag gac gaa gaa agg cta ctc aga gtg aat aaa tgt gcc ttt tct 5190
Lys Lys Asp Glu Glu Arg Leu Leu Arg Val Asn Lys Cys Ala Phe Ser
        1625                1630                1635

aag ttg cag aag ata tat gat act ttg tct aaa gat tta aac aat gaa 5238
Lys Leu Gln Lys Ile Tyr Asp Thr Leu Ser Lys Asp Leu Asn Asn Glu
    1640                1645                1650

cca att tcc cct att ggg ctt gag gag gat act ata att gct tcc aga 5286
Pro Ile Ser Pro Ile Gly Leu Glu Glu Asp Thr Ile Ile Ala Ser Arg
1655                1660                1665                1670

aag tca gat cat cca ata acg aag cac att agc ata gaa aat tct aaa 5334
Lys Ser Asp His Pro Ile Thr Lys His Ile Ser Ile Glu Asn Ser Lys
                1675                1680                1685

ttt aac agt aat ttg ctt gca cac cca gat att tgt tgt att agt gag 5382
Phe Asn Ser Asn Leu Leu Ala His Pro Asp Ile Cys Cys Ile Ser Glu
            1690                1695                1700

ata ttg gat cag gct gaa ttt gca gac ctt aaa aaa tta cag gat ctc 5430
Ile Leu Asp Gln Ala Glu Phe Ala Asp Leu Lys Lys Leu Gln Asp Leu
        1705                1710                1715

acc ttg aga tgt aca gat cac tta gaa att tta aaa aaa tac ttt cag 5478
Thr Leu Arg Cys Thr Asp His Leu Glu Ile Leu Lys Lys Tyr Phe Gln
    1720                1725                1730

atg cta caa gat aat aac atg gat aat att ttt atc aca gaa gaa aat 5526
Met Leu Gln Asp Asn Asn Met Asp Asn Ile Phe Ile Thr Glu Glu Asn
1735                1740                1745                1750

gtt tta gac gtg gtg ata aac cac agc cat gag gct atc att tta aag 5574
Val Leu Asp Val Val Ile Asn His Ser His Glu Ala Ile Ile Leu Lys
                1755                1760                1765

cct gaa gct att gaa atg tat att gaa atc gtc atg gtc tca gaa aca 5622

Pro Glu Ala Ile Glu Met Tyr Ile Glu Ile Val Met Val Ser Glu Thr
            1770                1775                1780

att cac ttt ctt aaa aac tca ata gca aag aaa cta gac aaa cag agg 5670
Ile His Phe Leu Lys Asn Ser Ile Ala Lys Lys Leu Asp Lys Gln Arg
        1785                1790                1795

ttt cga ggt atg ctt tgg ttt gat ttg tca ctt ctt cct gag ctg gtt 5718
Phe Arg Gly Met Leu Trp Phe Asp Leu Ser Leu Leu Pro Glu Leu Val
    1800                1805                1810

cag tgc caa gaa aaa atg gct tct ttt tca ttt ctt aaa gat aac tca 5766
Gln Cys Gln Glu Lys Met Ala Ser Phe Ser Phe Leu Lys Asp Asn Ser
1815                1820                1825                1830

aca gat gtt tgc ctt tgg aaa gtg ata gag act gct gtt tcc gaa ctt 5814
Thr Asp Val Cys Leu Trp Lys Val Ile Glu Thr Ala Val Ser Glu Leu
                1835                1840                1845

aag aaa gat ctg gat att atc tgg caa aat ata atg aaa gct gtt aaa 5862
Lys Lys Asp Leu Asp Ile Ile Trp Gln Asn Ile Met Lys Ala Val Lys
            1850                1855                1860

ttt gct cat atg cta ttc att tgc ttc ttc aag gag aac ttc aag gaa 5910
Phe Ala His Met Leu Phe Ile Cys Phe Phe Lys Glu Asn Phe Lys Glu
        1865                1870                1875

cct ttc aga aat aaa aaa gct tct gaa gaa gtc caa gta tta att cca 5958
Pro Phe Arg Asn Lys Lys Ala Ser Glu Glu Val Gln Val Leu Ile Pro
    1880                1885                1890

cat ata ttg acc ttt gtg cca tat ata gca tcc ata aat tat gga agc 6006
His Ile Leu Thr Phe Val Pro Tyr Ile Ala Ser Ile Asn Tyr Gly Ser
1895                1900                1905                1910

act gtg aca gag tta gaa tac aac tac aat caa ttt tct aca ctg ctg 6054
Thr Val Thr Glu Leu Glu Tyr Asn Tyr Asn Gln Phe Ser Thr Leu Leu
                1915                1920                1925

aag aat gta atg tct gcc cct agg aaa gat tta gga aaa atg gcc cac 6102
Lys Asn Val Met Ser Ala Pro Arg Lys Asp Leu Gly Lys Met Ala His
            1930                1935                1940

att agg aaa gtc atg aaa acg att gaa cat atg aag atg ata tgt act 6150
Ile Arg Lys Val Met Lys Thr Ile Glu His Met Lys Met Ile Cys Thr
        1945                1950                1955

aaa aat gct gaa cta acc att tcc ttt ttc cta tgc caa atg ctg tat 6198
Lys Asn Ala Glu Leu Thr Ile Ser Phe Phe Leu Cys Gln Met Leu Tyr
    1960                1965                1970

aac aga agg aag att tta cag ctg aag aga aaa gaa aaa atg aat att 6246
Asn Arg Arg Lys Ile Leu Gln Leu Lys Arg Lys Glu Lys Met Asn Ile
1975                1980                1985                1990

cat att gta aaa cct ggg gaa aat aac aat aaa ttt agt att tct acg 6294
His Ile Val Lys Pro Gly Glu Asn Asn Asn Lys Phe Ser Ile Ser Thr
                1995                2000                2005

atg ttg ccc cca gta tca gag tgc ata aac aaa aac atc tca aat tcc 6342
Met Leu Pro Pro Val Ser Glu Cys Ile Asn Lys Asn Ile Ser Asn Ser
            2010                2015                2020

tct aaa aaa cga ccg agc act gta gac aaa tgt gaa gac tct cag gaa 6390
Ser Lys Lys Arg Pro Ser Thr Val Asp Lys Cys Glu Asp Ser Gln Glu
        2025                2030                2035

cag cag caa gat act act gtt tcc agt tgt aaa aag cta aag gta gac 6438
Gln Gln Gln Asp Thr Thr Val Ser Ser Cys Lys Lys Leu Lys Val Asp
    2040                2045                2050

atg aaa gat gtc aca aaa atc aac aga gaa aag gca aca ttc aag cat 6486
Met Lys Asp Val Thr Lys Ile Asn Arg Glu Lys Ala Thr Phe Lys His
2055                2060                2065                2070

cca agg act aca gga tct cat ccc aaa agc gaa aac aaa ata gta cca 6534
Pro Arg Thr Thr Gly Ser His Pro Lys Ser Glu Asn Lys Ile Val Pro
                2075                2080                2085

agt tca tgt gac agt ctg aaa aga aat cat tta acg cca aaa aag gtt 6582
Ser Ser Cys Asp Ser Leu Lys Arg Asn His Leu Thr Pro Lys Lys Val
            2090                2095                2100

gaa atg caa aga tca cta cct ggc tca ctt tta ccc tta gag aac cca 6630

Glu Met Gln Arg Ser Leu Pro Gly Ser Leu Leu Pro Leu Glu Asn Pro
        2105                2110                2115

aaa gac act tgc gca tca aag tcg gaa agc aaa ata gac tta act gtt 6678
Lys Asp Thr Cys Ala Ser Lys Ser Glu Ser Lys Ile Asp Leu Thr Val
    2120                2125                2130

tca tct gat cac ttc agt gga caa cag gaa aat tta aat agc atg aag 6726
Ser Ser Asp His Phe Ser Gly Gln Gln Glu Asn Leu Asn Ser Met Lys
2135                2140                2145                2150

aaa aga aat gtg aac ttc agt gct gct gaa aca aaa agt gat aag aaa 6774
Lys Arg Asn Val Asn Phe Ser Ala Ala Glu Thr Lys Ser Asp Lys Lys
                2155                2160                2165

gat tgt gct gct ttt gca att tgt gac caa aaa agt gta cat ggc aca 6822
Asp Cys Ala Ala Phe Ala Ile Cys Asp Gln Lys Ser Val His Gly Thr
            2170                2175                2180

ttt tca cca gac cat ggg aca ctt ttg cag aaa ttt ctt aaa aat tcc 6870
Phe Ser Pro Asp His Gly Thr Leu Leu Gln Lys Phe Leu Lys Asn Ser
        2185                2190                2195

cac agg gtt gtt tat aat agc aat ata cat aca cat ttg gct agt act 6918
His Arg Val Val Tyr Asn Ser Asn Ile His Thr His Leu Ala Ser Thr
    2200                2205                2210

agg tgaataggaa aataaatcat gctgtatgta tacaataaga ggtcaagttg 6971
Arg
2215

ccaataaatt attactgtta atgttctggg aaatgctgaa actatgctaa gtgggggaga 7031

gggaaagcag gtattgcagt tttgtagtga agatgggctt tggagtcata tctgagatgt 7091

aagtagcagc ttttaaattc tagctatgac cctgtgcaga tcacttaact tttgagtgtc 7151

aggatgtttg gaaggacaag acaggaaagt gttttaatac agggttcagt atttagtaag 7211

ccttcaataa gtgatattac atacatgtaa taaaaactaa gcattgaaag gactaggaaa 7271

aatgcatcaa atgttagttc tgcaggttat agaattatac ttttatgtaa ttttaaaatt 7331

tcagttatga acatgtttgt ccattacaaa cttataatag ctgaaaaaca gggttgtgag 7391

aacagcttca gttaaaattg tcctgtctag ttttgaatat tcaagaacag aaagccttt 7450

## Description

### Technical Field

The present invention relates to polynucleotides, polypeptides, remedies for cancer, and vaccines used for diagnosis of autoimmune diseases such as graft versus host disease (hereafter referred as "GVHD") and graft versus leukemia (hereafter referred as "GVL"), and for treatment and prevention of cancers such as acute myelogenous leukemia (hereafter referred as "AML") and chronic myelogenous leukemia (hereafter referred as "CML").

### Background Art

GVHD, which is a kind of autoimmune disease characterized by graft versus host reaction, is a disease in which lymphatic system immunocytes (especially, T cells) of a transplanted donor immunologically attack tissue cells of a recipient (a host) whose gene organization differs, and the tissues of the recipient receive damages. The GVHD frequently occurs after bone marrow transplantation (BMT) for an AML or a CML patient due to the difference of human leukocyte antigen (HLA) between the donor and the recipient. On the other hand, GVL is a kind of autoimmune disease, and is caused by lymphatic system immunocytes of the donor immunologically attacking leukemia cells in the recipient after bone marrow transplantation for the AML or the CML patient.

Recently, an approach in which cDNA (complementary DNA) library of a human cell or a human cell line is screened using serum of these autoimmune disease patients and then oncogene and target gene of autoimmune disease are identified, has been noticed, and many genes have ever been identified. A polypeptide, which is a product of these genes, includes an antigen that is at least recognized by helper T cells in a living body of the patient. Therefore, the polypeptide has high possibility of becoming a key that causes cancers such as AML and CML and autoimmune diseases such as GVHD and GVL.

### DISCLOSURE OF THE INVENTION

The objectives of the present invention are to provide polynucleotides, polypeptides, remedies for cancer and vaccines used for diagnosis of autoimmune diseases such as GVHD and GVL and for treatment and prevention of AML or CML.

To achieve the above objectives, the first aspect of the present invention includes a polynucleotide comprising a base sequence that is identical or substantially identical with a base sequence represented by SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, and SEQ ID NO: 10.

Another aspect of the present invention includes a recombinant vector containing the above-mentioned polynucleotide.

The further aspect of the present invention includes a transformant containing the above-mentioned recombinant vector.

The polynucleotide of the present invention may be used with labeled with radioisotope, enzyme, or fluorochrome.

The further aspect of the present invention includes a polypeptide comprising an amino acid sequence that is identical or substantially identical with an amino acid sequence encoded by the above-mentioned polynucleotide.

The further aspect of the present invention includes an antibody against the polypeptide of the present invention.

The further aspect of the present invention includes a hybridoma producing an antibody of the present invention.

The further aspect of the present invention includes a diagnostic product for autoimmune diseases containing a polypeptide of the present invention.

The further aspect of the present invention includes a diagnostic kit of autoimmune diseases, which contains the polypeptide of the present invention, for screening serum of a GVHD or a GVL patient.

The further aspect of the present invention includes a kit for examining diseases, which contains the antibody of the present invention, for analyzing living tissues of an AML, a CML, a GVHD, or a GVL patient.

The further aspect of the present invention includes a remedy for cancer, which contains the polynucleotide of the present invention or the polypeptide of the present invention, for treating a cancer by strengthening the defense mechanism of a living body against cancer cells.

The further aspect of the present invention includes a vaccine, which contains the polynucleotide of the present invention or the polypeptide of the present invention, for preventing a cancer by allowing a living body to acquire the defense mechanism of the living body against cancer cells.

The further aspect of the present invention includes a DNA chip containing the above-mentioned polynucleotide.

### BEST MODE FOR CARRYING OUT THE INVENTION

The present embodiments will be described below in detail.

The polynucleotide of the present embodiment includes a base sequence represented by SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO:. 8, SEQ ID NO: 9, and SEQ ID NO: 10. The polynucleotide represented by SEQ ID NO: 1 to SEQ ID NO: 10 is a part of human genome cloned from human leukemia K562 cells.

The polynucleotide including a base sequence represented by SEQ ID NO: 1 and SEQ ID NO: 2 (hereafter referred as "pn1" and "pn2") encodes a gene product specifically causing antigen-antibody reaction with an antibody contained in serum of an AML patient who developed GVHD after the bone marrow transplantation and is still in a condition of developing GVHD. The polynucleotide including a base sequence represented by each of SEQ ID NO: 3 to SEQ ID NO: 8 (hereafter referred as "pn3" to "pn8") encodes a gene product specifically causing antigen-antibody reaction with an antibody contained in serum of a CML patient.

The polynucleotide including a base sequence represented by SEQ ID NO: 9 (hereafter referred as "pn9") encodes a continuous base sequence of a genome of K562 cells including a base sequence represented by SEQ ID NO: 1. 1 - 1788 in the sequence is likely to encode open reading frame (ORF). Here, the pn9 is predicted to be confirmed an initiation codon at a position where it slightly extends to an N-terminal side. The polynucleotide including a base sequence represented by SEQ ID NO: 10 (hereafter referred as "pn10") encodes a continuous base sequence of a genome of K562 cells including a base sequence represented by SEQ ID NO: 2. 277 - 6921 in the sequence is likely to encode open reading frame (ORF).

The polynucleotide of the present embodiment includes a base sequence that is substantially identical with a base sequence represented by SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, and SEQ ID NO: 10.
"a base sequence that is substantially identical with" is an encoded base sequence similar to a base sequence represented by the SEQ ID NOs. having at least one of slight differences selected from deletion, addition, and substitution of partial bases, and has preferably more than 80 % homology, more preferably more than 90 % homology, and even more preferably more than 95 % homology. Alternatively, it contains preferably more than 50 %, more preferably more than 75 %, and even more preferably more than 95 % of base sequences with preferably more than 80 % homology, more preferably more than 90 % homology, and even more preferably more than 95 % homology with respect to the overall length of a base sequence represented by each of the SEQ ID Nos.

A polynucleotide of the present embodiment may be a polynucleotide fragment or an oligonucleotide fragment obtained by cutting the above-mentioned polynucleotide using various restriction enzymes such as endonuclease, exonuclease, and the like. The polynucleotide fragment or the oligonucleotide fragment may preferably have a length of more than 20 bases, more preferably more than 50 bases, and even more preferably more than 100 bases, and especially preferably more than 500 bases.

In addition, the substantially identical base sequence may preferably encode an identical or a substantially identical amino acid sequence. "an amino acid sequence that is substantially identical with" is an encoded amino acid sequence having at least one of slight differences selected from deletion, addition, and substitution of partial amino acids, and has preferably more than 80 % homology, more preferably more than 90 % homology, even more preferably more than 95 % homology, and most preferably more than 98% homology.

The polynucleotide may be stored and amplified in the condition that it is inserted in the predetermined position of a widely used vector in the form of double strand DNA. As the above-mentioned vectors, for example, plasmid vectors derived from *Escherichia coli,* yeast, or *Bacillus subtilis,* bacteriophage vectors such as phage λ, or virus vectors such as a retrovirus, an adenovirus, a vaccinia virus, and a baculovirus may preferably be used.

When the polynucleotide is amplified, a vector including such polynucleotide is initially constructed. Next, the vector is incorporated into an appropriate host cell to produce a transformant, and the transformant is proliferated to amplify the polynucleotide. The above-mentioned host cell may include, for example, bacteria (genus *Escherichia,* genus *Bacillus*)*,* yeasts, insect cells, animal cells, or mammalian cells. The vectors and the polynucleotides may easily and massively be produced using these transformants, and then gene product of the polynucleotide may also easily and massively be produced.

Alternatively, the polynucleotide may massively be obtained by extracting total RNA or messenger RNA (mRNA) fraction from K562 cells, other human cell line, human culture cells, or human tissues, and directly amplifying the extracted solution using RT-PCR method(Reverse Transcriptase Polymerase Chain Reaction Method). Alternatively, the polynucleotide may be obtained by artificial synthesis using an automated DNA synthesis machine.

On the other hand, the polynucleotide may be used as an antisense DNA or an antisense RNA that is a complementary sequence with respect to a base sequence that is normally expressed as mRNA. More specifically, when at least part of the base sequence of the polynucleotide has a sequence specific for cancer cells of an AML or a CML patient, the specific sequence or a neighboring sequence of the specific sequence including the specific sequence may be used for diagnosis of AML or CML, and thus may assist subsequent treatment. That is, the above-mentioned sequence may be used for a diagnostic probe for well-known methods such as Southern hybridization method, Northern hybridization method, and PCR (Polymerase Chain Reaction) method, after the cancer cells are obtained from the AML or the CML patient. Tissues obtained from the above-mentioned patient may also be used for analyzing expression site or expression condition of the polynucleotide including the above-mentioned sequence, and localization on the human chromosome using in situ hybridization method. Here, the polynucleotide may normally used with labeled with radioisotope, enzyme, or fluorochrome.

The antisense DNA and the antisense RNA may be used as a remedy for alleviating symptom of autoimmune diseases such as GVHD, GVL, and the like by suppressing the expression level of mRNA of the polynucleotides recognized as an antigen so that the amount of the resultant gene product decreases. When a polynucleotide is one that encodes a polypeptide relating to crisis of a cancer, it may be used as a remedy for cancer for suppressing proliferation of cancer cells by suppressing the production amount of the gene product of the polynucleotide.

The antisense DNA or the antisense RNA may preferably be administered into a human body independently or with a form in which it is inserted into a retrovirus vector, an adenovirus vector, or an adeno-associated virus vector.

The polypeptide of the present embodiment includes an amino acid that is identical or a substantially identical with an amino acid encoded by the above-mentioned polynucleotide. That is, it includes an amino acid that is identical or a substantially identical with an amino acid represented by SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, and SEQ ID NO: 10.
"an amino acid sequence that is substantially identical with" is an encoded amino acid sequence similar to an amino acid sequence having at least one of slight differences selected from deletion, addition, and substitution of partial amino acids. It is encoded with an amino acid sequence having preferably more than 80 % homology, more preferably more than 90 % homology, even more preferably more than 95 % homology, and most preferably more than 98 % homology.

More specifically, when an amino acid sequence is an encoded one having slight differences through the substitution, the property of the amino acid to be substituted may preferably be similar to that of a substituted amino acid. As an example of such pairs of an amino acid to be substituted and a substituted amino acid, glycine and alanine; valine, leucine, and isoleucine; proline, phenylalanine, and tryptophan; aspartic acid and glutamic acid; asparagine and glutamine; serine, threonine, and tyrosine; or lysine, arginine, and histidine may be included. In these substitutions, tertiary structure of a polypeptide is similar in many cases. Therefore, there is high. possibility of being recognized to an identical antibody, and there is high possibility of working in a living body of a patient as an antigen that causes autoimmune disease such as GVHD and GVL.

The polypeptide of the present embodiment may be a polypeptide fragment or an oligopeptide fragment obtained by cutting the polypeptide using a proteolytic enzyme such as protease, proteinase, endopeptidase, exopeptidase, and the like. These polypeptide fragments or oligopeptide fragments have high possibility of working as an antigen that causes autoimmune diseases in a living body of a patient.

In general, the polypeptides may easily and massively be obtained by compulsorily expressing the polynucleotide inserted into the transformant. Alternatively, it may be obtained by artificially synthesizing with solid phase synthesis method or liquid phase synthesis method. Alternatively, it may also be obtained by fractioning and purifying protein extraction fraction extracted from K562 cells, other human cell line, human culture cells, or human tissues using affinity chromatography carrier with which an antibody against the polypeptide, which is produced in advance, is combined.

The antibody may include a monoclonal antibody or a polyclonal antibody. The polyclonal antibody may be preferable for increasing diagnosis sensitivity, while the monoclonal antibody may be preferable for detailed diagnosis or analysis. The polyclonal antibody is generally obtained by multiply immunizing an immune animal such as a goat, a rabbit, cattle, and a horse with the polypeptide together with an adjuvant if necessary, and collecting serum from the immune animal. The monoclonal antibody is generally obtained by multiply immunizing an immune animal such as a mouse, a rat, a goat, and a rabbit with the polypeptide together with an adjuvant if necessary, and collecting B cells from the immune animal, cell-fusing the B cells with myeloma cells to produce hybridoma, and obtaining culture supernatant of a single hybridoma that was cloned.

These antibodies (primary antibodies) may be used as a diagnostic tool or an analyzing tool for analyzing localization where the polypeptide is expressed in tissues or cells (i.e., immunohistochemical staining) with secondary antibodies that are labeled with radioisotope, enzyme, fluorochrome, or the like. Also, it may be used for identification of an antigen that causes GVHD or GVL, or quantitation of the expression level of the polypeptide. When the polypeptide includes physiological activity such as enzymes and the antibody is a neutralizing antibody for such polypeptide, the antibody may be used, for example, as a remedy for cancer since it inhibits the physiological activity of the polypeptide that works as an antigen.

The remedy for cancer of the present embodiment contains the above-mentioned polynucleotide or polypeptide, and immunologically treats the cancer by strengthening the defense mechanism of a living body against cancer cells in the living body of a cancer patient. The defense mechanism of the living body includes at least one of immune systems selected from antibody-mediated immunity targeting a gene product from the polynucleotide or the polypeptide, and cell-mediated immunity. The cancer cell exposes on the surface of cells in a form in which it recognizes the target as an immunologically not-self, and the main target of the remedy for cancer includes cancer cells of an AML or a CML patient.

The vaccine of the present embodiment contains the above-mentioned polynucleotide or polypeptide, and immunologically prevents a cancer by allowing a living body to acquire the defense mechanism of the living body against cancer cells. The defense mechanism of the living body includes an identical immune system with that for the remedy for cancer. The vaccine may be used for acquiring the preliminary defense mechanism of a living body to inhibit the occurrence of cancer cells that are exposed on the surface of a cell in the form in which the vaccine recognizes the target as an immunologically not-self. It may mainly be used for preventing morbidity for AML or CML.

The remedy for cancer or the vaccine of the present embodiment may be used as a mixed remedy for cancer or a mixed vaccine by appropriately mixing at least one selected from the above-mentioned polynucleotides with at least one selected from the above-mentioned polypeptides. In addition, it is preferable that the mixed remedy for cancer may simultaneously contain a polypeptide as a tumor antigen that is included in cancer cells, which is a target for the treatment, and a polynucleotide that encodes the polypeptide.

The remedy for cancer or the vaccine may be used, for example, in the dosage form of an injection. The injection may contain at least one selected from the polynucleotide and the polypeptide in an isotonic solution containing physiological saline, grape sugar and an adjuvant (e.g., sorbitol and mannitol). If necessary, the injection may include at least one selected from solubilizing agent, buffer for holding pH of a solution constantly, stabilizer (antioxidant) and preservative for improving the stability, and an analgesic agent for alleviating pain during the injection.

As the solubilizing agent, for example, alcohols such as ethanol, polyethylene glycol, and propylene glycol; nonionic surfactants; fats and oils such as sesame oil and soybean oil; benzyl benzoate; and benzyl alcohol may preferably be used. Preferably, the stabilizer, the preservative and the analgesic agent listed in Japanese pharmacopoeia may be used. In addition, at least one selected from various vitamins, well-known pharmaceutical formulations used for the cancer treatment such as interferons α to γ, physiologically active substances for activating the immune system such as interleukin 1 (α, β), interleukins 2 to 8 and other cytokines may preferably be contained.

The operations of the above embodiments will now be described.

When a patient of autoimmune disease such as GVHD or GVL or a person who is suspicious for the autoimmune disease (hereafter, both referred as "a person to be diagnosed") is diagnosed using the polypeptide, blood from the person to be diagnosed is initially obtained to fractionate its serum. Next, the diagnosis of the autoimmune disease is carried out through examining the antigen-antibody reaction between antibody contained in the serum and the polypeptide.

Here, for example, quanlitative reactions such as immunoprecipitation reaction, double gel diffusion method, and immunoelectrophoresis; and quanitative reactions such as quantitative precipitation reaction, radioimmunoassay, and enzyme-linked immunosorbent assay may be used as the antigen-antibody reaction. When the polypeptide or the protein including the polypeptide has physiological activity such as enzyme, it is possible to confirm the specific antigen-antibody reaction by examining the reduction of the physiological activity and also to prove that the antigen contained in the serum is a neutralizing antibody. In addition, after confirming the existence of the antibody against the polypeptide in the serum of the person to be diagnosed, the tissue is obtained from the person to be diagnosed and the localization of the antigen may immunohistologically be analyzed using an immunohistochemical staining method.

On the other hand, when the injection containing the polynucleotide as a remedy for cancer is administered into a living body of the AML or the CML patient, the polynucleotide is incorporated into the cells of the patient, is translated, and a resultant gene product is produced. At least part of the produced gene product is processed within cells in various manners and then is fragmented small. Subsequently, the fragmented product is combined with a class I or a class II HLA protein that exists in the cell membrane surface, and then is recognized by T cells as a tumor antigen.

When the fragmented gene product is combined with the class I HLA protein, cytotoxic T lymphocytes (CTL) in the cancer patient's body recognize the fragment with the tumor antigen, and kill the cells. In addition, this stimulates the CTL, and the CTL is rapidly cell-divided to proliferate. Subsequently, the increased number of CTL after the proliferation circulates in a living body of the patient, finds cells presenting the similar fragment on the HLA protein, i.e., cancer cells, and then kills these cells.

When the fragmented gene product is combined with the class II HLA protein, helper T cells existing in the patient's body recognize the fragment with a tumor antigen. The information that the fragment is the tumor antigen stimulates B cells to produce antibodies, and transmits the proliferative stimulus to the CTL. Subsequently, the B cells are rapidly cell-divided to proliferate, and then the antibodies that are more specifically combined with the fragment are massively produced. Then, these antibodies are specifically combined with the fragment or the gene product of the polynucleotide, and then immunological attack against the fragment and the gene product by phagocytes such as macrophages and complements of the patient's body may be promoted. When the gene product has physiological activity, the combination of the antibody neutralizes its physiological activity.

When the injection containing the polypeptide is administered into a living body of the AML or the CML patient as a remedy for cancer, most of the polypeptides are recognized by helper T cells and B cells as a tumor antigen, and a part of the polypeptides are incorporated into cells (mainly, the phagocytes) of the patient's body, and are presented on the cell surface together with class I or class II HLA protein. As a result, as described above, cancer cells are dead through the attack by the immune system including the CTLs, the B cells, the phagocytes, and complements.

In addition, when the mixed remedy for cancer is administered into a living body of the AML or the CML patient, cell group of immune system is activated against both the gene product of the polynucleotide and the polypeptide, and then additive effect or synergistic effect is displayed. On the other hand, a part of cell group of the immune system (helper T cells, CTLs, B cells) activated by the administration of the remedy for cancer keeps circulating in the living body of the cancer patient as a memory cell, and prevent survived cancer cells or cancer cells that present a similar fragment from proliferating or developing again.

When a vaccine containing the polynucleotide (a DNA vaccine) is administered into a living body of a human in the form of an injection, the polynucleotide is incorporated in cells of the human's body, and then is translated. As a result, a gene product of the polynucleotide is expressed. As described above, T cells recognize the resultant gene product as an antigen together with class I or class II HLA protein. Therefore, the process of a series of weak immune system activation is passed to exclude the antigen, and finally memory cells are produced. When the vaccine containing the polypeptide is administered into the living body of the human in the form of an injection, the process of a series of weak immune system activation is also passed, and finally memory cells are produced.

In addition, when the mixed vaccine is administered into a living body of the AML or the CML patient, cell group of immune system is activated against both the gene product of the polynucleotide and the polypeptide, and then additive effect or synergistic effect is displayed. It is preferable that these vaccines are multiply administered at a predetermined interval. As a result, the specificity of the memory cells with respect to the fragment or the gene product is further increased, and the c of cancer cells presenting the similar fragment is further strongly inhibited.

The above described embodiments have the following effects.

The polynucleotide of the present invention includes a base sequence that is identical or substantially identical with a base sequence represented by SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, and SEQ ID NO: 10. It is possible to treat a cancer by administering these polynucleotides to an AML or a CML patient, and then strengthening the defense mechanism of a living body of the patient against the cancer cells. In addition, it is possible to prevent development and proliferation of the cancer cells by administering these polynucleotides to a person other than the patient to have the person acquire defense mechanism of a living body against the cancer cells that causes AML or CML.

The recombinant vector of the present invention is a recombinant vector containing a polynucleotide of the present invention. The recombinant vector allows the polynucleotide to be preserved easily and stably.

The transformant of the present invention is a transformant containing the recombinant vector of the present invention. The transformant allows the polynucleotide and the vector to be easily amplified to obtain them.

The polynucleotide of the present invention may be labeled with radioisotope, enzyme or fluorochrome. It is possible to analyze expression of the polynucleotide in the living tissues with high sensitivity, and to assist diagnosis and treatment.

The polypeptide of the present invention includes an amino acid sequence that is identical or substantially identical with an amino acid sequence encoded by the above-mentioned polynucleotide. It is possible to treat a cancer by administering these polypeptides to an AML or a CML patient, and then strengthening the defense mechanism of a living body of the patient against the cancer cells. In addition, it is possible to prevent development and proliferation of the cancer cells by administering these polynucleotides to a person other than the patient to have the person acquire defense mechanism of a living body against the cancer cells that causes AML or CML. Further, the diagnosis of GVHD or GVL may easily be carried out by examining whether the antigen-antibody reaction occurs against serum of a person to be diagnosed using these polypeptides.

The antibody of the present invention is an antibody against the polypeptide of the present invention. The use of the antibody may easily analyze the localization of the polypeptide in the living tissues, and thus may assist diagnosis and treatment.

The hybridoma of the present invention is a hybridoma producing an antibody of the present invention. Monoclonal antibodies, which are able to analyze the localization of the polypeptides in a living body and to assist diagnosis and treatment, may easily and massively be obtained from the hybridoma.

The diagnostic product for autoimmune diseases of the present invention contains the polypeptide of the present invention, and examines antigen-antibody reaction against an antibody contained in serum of a person to be diagnosed. The diagnostic product may easily carry out the diagnosis for GVHD or GVL.

The diagnostic kit of autoimmune diseases of the present invention contains the polypeptide of the present invention, and screens serum of a GVHD or a GVL patient. The diagnostic kit may easily carry out the diagnosis for GVHD or GVL.

The examining kit of the present invention contains the antibody of the present invention, and may analyze living tissues of an AML, a CML, a GVHD, or a GVL patient. It is possible to easily analyze the localization of the polypeptides in a living body, and may assist diagnosis and treatment by using the examining kit.

The remedy for cancer of the present invention contains at least one selected from the polynucleotide and the polypeptide, and treat cancer by strengthening the defense mechanism of a living body against cancer cells. Therefore, the administration of the remedy for cancer of the present invention may positively induce GVL condition in the living body of the AML or the CML patient, and thus may effectively treat cancer. In addition, the remedy for cancer may be composed of a mixed remedy containing at least one of the polynucleotides and at least one of the polypeptides. This composition may further strengthen the defense mechanism of the living body.

The vaccine of the present invention contains at least one selected from the polynucleotides and the polypeptides, and makes a living body to acquire the defense mechanism of the living body against cancer cells to prevent the onset and increasing cancer cells. The vaccine may mainly be administered into a person who does not get AML or CML or a person who is likely to genetically get the AML or the CML to effectively prevent the person from getting the AML or the CML. In addition, the vaccine may be composed of a mixed vaccine containing at least one of the polynucleotides and at least one of the polypeptides. This composition may further increase the preventive effect.

The DNA chip of the present invention is a DNA chip containing the polynucleotide of the present invention. When the DNA chip is used, a monitoring of gene expression may easily be carried out.

### Examples

Examples that embody the above embodiments will now be described.

### (Screening of cDNA library)

Patient A (a woman of 42 year-old) of the high risk case, who is diagnosed with AML M1 Ph1+, was transplanted bone marrow obtained from her elder sister, who matches HLA, in her primary remission phase. The patient A retained the complete remission for a while. However, one and half years later, the reduction of myodynamia in her whole body with muscular atrophy appeared. For this symptom, the treatment with an immunosuppressant and the like was carried out. However, she finally died of the respiratory insufficiency due to the chronic GVHD.

Cell lysates of various cell lines were analyzed with Western blotting method using mixed serum in which the serum of patient A when the muscular atrophy appeared was mixed with the serum of CML patients B to D. As a result, it was proven that the mixed serum caused strongly specific antigen-antibody reaction in human leukemia cell K562 cells.

Next, cDNA library was produced from the K562 cells using a random primer (a hexamer). On about 10⁶ clones contained in this library, a phage λ, which contains the cDNA encoding the polypeptide that combines with the antibody in the mixed serum, was screened using serological analysis of antigens by recombinant cDNA expression cloning method (SEREX method). Ten kinds of clones that finally reacted strong-positively were isolated and identified.

### (Characterization)

The obtained ten kinds of clones were determined their base sequences. As a result, eight kinds of base sequences represented by SEQ ID NO: 1 to SEQ ID No. 8 (i.e., pn1 to pn8) were determined. Here, remaining two kinds of clones overlap with pn3 and pn7, respectively. Here, the clone that overlapped with pn3 (reacting with the serum of patient B) reacted with the serum of patient C. These results and examining results of origin of the serum (patient) in which the gene product of each clone combined are shown in Table 1.

**Table 1**

| | Homology Search Result | Disease name or Symptom | Patient |
|---|---|---|---|
| pn1 | novel | AML, GVHD after BMT | A |
| pn2 | novel | AML, GVHD after BMT | A |
| pn3 | novel | CML | B, C |
| pn4 | novel | CML | C |
| pn5 | novel (kif1a) | CML | C |
| pn6 | LEDGF | CML | C |
| pn7 | Sip1 | CML | C |
| pn8 | HSP70 | CML | D |

### (Cloning of pn9 and pn10)

An oligo-DNA primer that complementarily combines with both ends of each of pn1 and pn2, respectively, was artificially synthesized. The cDNA library was screened using both primers, while each of pn1 and pn2 was extended to the 5' end side and the 3' end side. Finally, a base sequence that is regarded as ORF including pn1 or pn2, respectively, i.e., base sequence of pn9 or pn10, was determined.

### (Homology Search)

Homology search regarding the base sequences of pn1 to pn10 with respect to base sequences of well known nucleic acids that were already registered on the nucleic acid sequence databases was carried out using BLASTN(GENBANK). Regarding some clones, homology search regarding the amino acid sequences encoded by the base sequences with respect to amino acid sequences of well known polypeptides that were already registered on the amino acid sequence databases was carried out using BLASTP(Swiss Plot). In addition, regarding the amino acid sequences, at least one kind of searches selected from the search of the localization using RESORT and the search of the protein motif using MOTIF was carried out.

Useful information extracted from these search results was shown at the column of "Homology Search Result" of Table 1. It was predicted that pn3 and pn4 have encoded an amino acid sequence with zinc finger domain, and pn5 has encoded an amino acid sequence homologous with the kinesin. In addition, it was predicted that pn9 and pn10 have encoded a protein localized in the nucleus since these have high hydrophobic sites and a plurality of phosphorylation sites.

In the column of "Homology Search Result" shown in Table 1, "novel" refers that a base sequence and an amino acid sequence with specifically high homology were not confirmed, and pn5 has slightly high homologous with kinesin-like protein (kifla). In Table 1, "LEDGF" refers human epithelium lentis derived growth factor, "Sip1" refers human splicing factor Sip1, and "HSP70" refers 70kD (Dalton) heat shock protein.

### (Verification of reactivity for normal human serum)

For the polypeptide produced by phage λ containing each polynucleotide of pn1 to pn10, antigen-antibody reaction was carried out using mixed serum that was obtained by mixing serum obtained from a plurality of healthy persons. The antigen-antibody reaction was not confirmed for each of clones.

At least one kind of polynucleotides selected from pn1 to pn10 may be used for producing a DNA chip. By using the DNA chip, a monitoring of gene expression for the polynucleotide of each of pn1 to pn10 may easily be carried out.

The polypeptide of the present invention may be used for a method of diagnosing autoimmune diseases characterized by using the polypeptide for screening serum of a GVHD or a GVL patient. By using the polypeptide, the diagnosis for GVHD or GVL may easily be carried out.

The antibody of the present invention may be used for a method of examining diseases characterized by analyzing tissues in a living body of an AML, a CML, a GVHD, or a GVL patient. The use of the antibody may easily analyze the localization of the polypeptide in a living body, and thus may assist diagnosis and treatment.

## Claims

1. A polynucleotide comprising a base sequence that is identical or substantially identical with a base sequence represented by SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, and SEQ ID NO: 10.

2. A recombinant vector containing the polynucleotide according to claim 1.

3. A transformant containing the recombinant vector according to claim 2.

4. The polynucleotide according to claim 1 labeled with radioisotope, enzyme, or fluorochrome.

5. A polypeptide comprising an amino acid sequence that is identical or substantially identical with an amino acid sequence encoded by the polynucleotide according to claim 1.

6. An antibody against the polypeptide according to claim 5.

7. A hybridoma producing the antibody according to claim 6.

8. A diagnostic product for autoimmune diseases containing the polypeptide according to claim 5.

9. A diagnostic kit of autoimmune diseases for screening serum of a GVHD or a GVL patient, wherein the diagnostic kit contains the polypeptide according to claim 5.

10. A kit for examining diseases for analyzing living tissues of an AML, a CML, a GVHD, or a GVL patient, wherein the kit contains the antibody according to claim 6.

11. A remedy for cancer for treating a cancer by strengthening the defense mechanism of a living body against cancer cells, wherein the remedy contains the polynucleotide according to claim 1.

12. A remedy for cancer for treating a cancer by strengthening the defense mechanism of a living body against cancer cells, wherein the remedy contains the polypeptide according to claim 5.

13. A remedy for cancer for treating a cancer by strengthening the defense mechanism of a living body against cancer cells, wherein the remedy contains the polynucleotide according to claim 1 and the polypeptide including an amino acid sequence that is identical or substantially identical with an amino acid sequence encoded by said polynucleotide.

14. The remedy for cancer according to any one of claims 11 to 13, wherein the remedy is prepared as an injection.

15. A vaccine for preventing development of a cancer by allowing a living body to acquire the defense mechanism of the living body against cancer cells, wherein the vaccine contains the polynucleotide according to claim 1.

16. A vaccine for preventing development of a cancer by allowing a living body to acquire the defense mechanism of the living body against cancer cells, wherein the vaccine contains the polypeptide according to claim 5.

17. A vaccine for preventing development of a cancer by allowing a living body to acquire the defense mechanism of the living body against cancer cells, wherein the vaccine contains the polynucleotide according to claim 1 and the polypeptide including an amino acid sequence that is identical or substantially identical with an amino acid sequence encoded by said polynucleotide.

18. The vaccine according to any one of claims 15 to 17, wherein the vaccine is prepared as an injection.

19. A DNA chip containing the polynucleotide according to claim 1.
